(19) Europäisches Patentamt
European Patent Office
Office européen des brevets

(11) Veröffentlichungsnummer: **0 352 638 B1**

# (12) EUROPÄISCHE PATENTSCHRIFT

(45) Veröffentlichungstag der Patentschrift: **22.02.95**

(21) Anmeldenummer: **89113378.7**

(22) Anmeldetag: **21.07.89**

(51) Int. Cl.$^{6}$: **C07D 237/22**, C07D 405/12, C07D 413/12, C07D 409/12, C07D 401/12, C07D 403/12, A01N 43/58

## (54) 5-Amino-6-pyridazonderivate, Verfahren zu ihrer Herstellung und sie enthaltende herbizide Mittel.

(30) Priorität: **27.07.88 DE 3825468**

(43) Veröffentlichungstag der Anmeldung:
**31.01.90 Patentblatt 90/05**

(45) Bekanntmachung des Hinweises auf die Patenterteilung:
**22.02.95 Patentblatt 95/08**

(84) Benannte Vertragsstaaten:
**BE CH DE FR GB IT LI NL**

(56) Entgegenhaltungen:
**DE-A- 1 695 840**

**CHEMICAL ABSTRACTS, Band 66, Nr. 11, 13. März 1967, Seiten 4402-4403, Zusammenfassung Nr. 46388y, Columbus, Ohio, US; T. NAKAGOME et al.: "Synthesis of pyridazine derivatives. XIII. Synthesis of N1-(2-methyl-3-oxo-2,3-dihydro-4-pyridazinyl-)sulfanil-amide derivatives", & CHEM. PHARM. BULL. (TOKYO) 14(10), 1082-1090(1966)**

(73) Patentinhaber: **BASF Aktiengesellschaft**
**Carl-Bosch-Strasse 38**
**D-67063 Ludwigshafen (DE)**

(72) Erfinder: **Wriede, Ulrich, Dr.**
**Birkenstrasse 7**
**D-6704 Mutterstadt (DE)**
Erfinder: **Wuerzer, Bruno, Dr.**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**
Erfinder: **Westphalen, Karl-Otto, Dr.**
**Mausbergweg 58**
**D-6720 Speyer (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft 5-Amino-6-pyridazone der Formeln Ia und/oder Ib

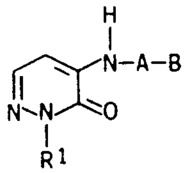

Ia

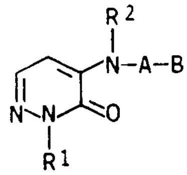

Ib

in denen die Substituenten folgende Bedeutung haben:

$R^1$
- eine $C_1$-$C_4$-Alkylgruppe, welche bis zu drei Halogenatome tragen kann,

$R^2$
- eine $C_1$-$C_4$-Alkylgruppe oder eine $C_3$-$C_6$-Alkenylgruppe, wobei diese Gruppen bis zu zwei Phenylreste tragen können oder
- eine $C_3$-$C_6$-Alkinylgruppe,

A -CO- oder $-SO_2-$

B
- eine $C_1$-$C_6$-Alkylgruppe, eine $C_2$-$C_6$-Alkenylgruppe oder eine $C_2$-$C_6$-Alkinylgruppe, wobei diese Gruppen bis zu drei der folgenden Reste tragen können: Halogenatome, $C_1$-$C_4$-Alkoxygruppen, $C_1$-$C_4$-Alkylthiogruppen und/oder Phenylreste,
- eine iso- oder heterocyclische $C_3$-$C_7$-Cycloalkylgruppe oder eine iso- oder heterocyclische $C_3$-$C_7$-Cycloalkenylgruppe, wobei diese Gruppen mit ein bis zwei Cycloalkylgruppen dieser Art, oder mit ein bis zwei Benzolkernen anelliert sein können, wobei die Gesamtzahl der Ringglieder 3 bis 16 beträgt und diese cyclischen Reste bis zu drei der folgenden Gruppen tragen können: Halogenatome, $C_1$-$C_4$-Alkylgruppen, $C_2$-$C_6$-Alkenylgruppen, $C_2$-$C_6$-Alkinylgruppen und/oder Phenylreste
- ein 1 bis 3 kerniger aromatischer oder heteroaromatischer Rest, welcher bis zu drei der folgenden Gruppen tragen kann: $C_1$-$C_4$-Alkylgruppen, $C_1$-$C_4$-Halogenalkylgruppen, $C_1$-$C_4$-Alkoxygruppen, $C_1$-$C_4$-Halogenalkoxygruppen, $C_1$-$C_4$-Alkylthiogruppen, $C_1$-$C_4$-Alkylsulfinylgruppen, $C_1$-$C_4$-Alkylsulfonylgruppen, Cyanogruppen, Nitrogruppen, Carbo-$C_1$-$C_4$-alkoxygruppen, N,N-Di-$C_1$-$C_4$-alkylcarbamidogruppen und/oder Halogenatome.

Weiterhin betrifft die Erfindung die Herstellung dieser Verbindungen sowie deren Verwendung als Herbizide.

Aus der DE-B 1 105 232 ist bekannt, daß N-substituierte 4-Amino-6-pyridazone herbizide Wirkung haben. Besonders für die Anwendung neben Kulturpflanzen z.B. im Nachauflaufverfahren sind jedoch Verbindungen wünschenswert, die bei geringerer Aufwandmenge höhere Selektivität aufweisen.

Entsprechend dieser Aufgabe wurden die eingangs definierten 5-Amino-6-pyridazonderivate Ia und Ib gefunden.

Ferner wurde gefunden, daß die Verbindungen Ia und Ib eine vorteilhafte herbizide Wirkung, besonders im Nachauflaufverfahren haben und gegen eine Reihe von Kulturpflanzen selektiv sind, und sich demgemäß gut als herbizide Mittel verwenden lassen.

Außerdem wurde ein Verfahren zur Herstellung der Verbindungen Ia und Ib gefunden.

Zur Herstellung der erfindungsgemäßen Verbindungen Ia setzt man ein Pyridazon II mit Hydrazin zum 5-Amino-6-pyridazon III um, welches anschließend mit einer Verbindung der Formel IV derivatisiert wird.

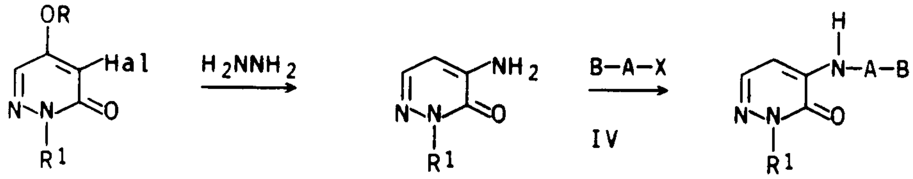


II III Ia

In der Formel II bedeutet R eine $C_1$-$C_4$-Alkylgruppe wie Methyl, Ethyl, n-Propyl, iso-Propyl und die vier isomeren Butylreste, besonders aber Methyl oder Ethyl und Hal Halogen wie Fluor, Chlor, Brom oder Jod, besonders aber Chlor oder Brom.

Die Reduktion mit Hydrazin wird vorzugsweise in wäßrigem Medium mit oder ohne Zusatz eines inerten Lösungsmittels wie z.B. Diethylether, Ethylenglykoldimethylether, Tetrahydrofuran, Methanol, Ethanol, iso-Propanol oder Dimethylsulfoxid, bei Temperaturen von 20 bis 120 °C, vorzugsweise 60 bis 100 °C, kontinuierlich oder diskontinuierlich, drucklos oder unter Druck (1 bis 10 bar) durchgeführt.

Die anschließende Umsetzung mit IV wird in an sich bekannter Weise (Houben-Weyl, Bd. 8, S. 655 ff und Bd. E5, S. 972 ff) vorzugsweise bei 0,5 bis 10 bar, besonders bei Normaldruck kontinuierlich oder diskontinuierlich bei Temperaturen von 0 bis 100 °C, vorzugsweise 20 °C bis 40 °C in einem Lösungsmittel sowie in Gegenwart einer Base durchgeführt.

In der Formel IV bedeutet X Halogen, z.B. Chlorid, Bromid und Jodid oder Carboxylat wie Acetat und Propionat, vorzugsweise jedoch Chlorid und Bromid. Diese Reste sind in aller Regel leicht abspaltbar.

Als Basen eignen sich tertiäre Amine wie Triethylamin, die Picoline, N,N-Dimethylanilin oder Pyridin, oder anorganische Basen wie z.B. Kaliumhydroxid, Natriumhydroxid, Kaliumcarbonat, Natriumbicarbonat, Bariumhydroxid und Natriumacetat. Bevorzugt werden Triethylamin und Pyridin.

Geeignete Lösungsmittel sind z.B. n-Hexan, Decalin, Toluol, Methylenchlorid, Diethylether, Tetrahydrofuran, Dioxan, Dimethylformamid, Methanol, Ethanol und iso-Propanol und entsprechende Gemische.

Man erhält die benötigten Pyridazone II beispielsweise nach den in der DE-OS 2 526 643 beschriebenen Bedingungen, indem man ein Dihalogen-6-pyridazon der allgemeinen Formel VI mit ungefähr der stöchiometrischen Menge eines Alkoholats der Formel VII, worin M ein Alkalimetallkation, insbesondere das Natrium oder Kaliumion und R ein Alkylrest mit 1 bis 4 Kohlenstoffatomen, insbesondere Methyl und Ethyl bedeuten, in Gegenwart eines organischen Lösungsmittels, vorzugsweise des entsprechenden Alkohols, bei einer Temperatur zwischen 0 und 100 °C, vorzugsweise 10 bis 40 °C, umsetzt.

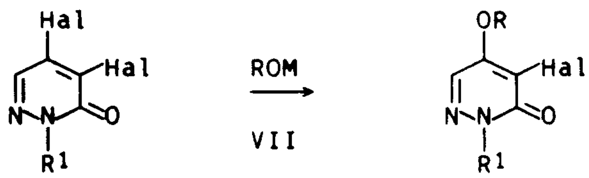


VI II

Die Verbindungen der Formel Ib erhält man in an sich bekannter Weise (Houben-Weyl, Bd. E5, S. 998 ff) durch Alkylierung, Alkenylierung oder Alkinylierung der Verbindungen Ia. Hierzu wird die Verbindung Ia mit einem Reagens der Formel V, worin Y eine leichtabspaltbare Gruppierung wie Halogen, z.B. Chlorid, Bromid und Jodid, Sulfonat wie Tosylat, Mesylat und Trifluormethylsulfonat oder Alkylsulfat, wie Methylsulfat und Ethylsulfat bedeutet, umgesetzt.

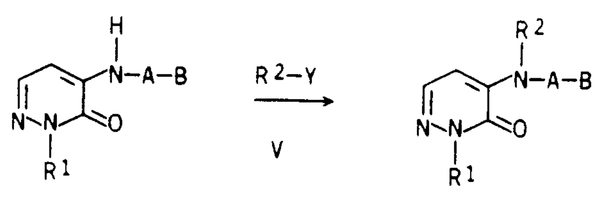


Ia Ib

Die Reaktion kann mit oder ohne Lösungsmittel, kontinuierlich oder diskontinuierlich, drucklos oder unter Druck (1 bis 10 bar) bei Temperaturen von 20 bis 180 °C, vorzugsweise bei 100 bis 160 °C, in Gegenwart einer starken Base durchgeführt werden.

Geeignete Basen sind hierfür z.B. Natriumhydrid, Kaliumhydrid, Lithiummethylat, Natriummethylat, Natriumethylat, Kalium-tert.-butylat, Lithiumamid, Natriumamid, Kaliumhydroxid oder Kaliumcarbonat. Bevorzugt werden Natriumhydrid, Natriummethylat und Kalium-tert.-butylat.

Als Lösungsmittel kommen z.B. n-Hexan, Decalin, Toluol, Diethylether, Ethylenglykoldimethylether, Dioxan, Dimethylformamid, Dimethylsulfoxid, Aceton, Methanol, Ethanol und iso-Propanol in Betracht.

Bevorzugt wird Dimethylformamid.

Im Hinblick auf die bestimmungsgemäße Verwendung der Verbindungen Ia und/oder Ib als Herbizide kommen folgende Reste in Betracht:

In der Bedeutung $R^1$ kommen als

- Alkylgruppen die Methyl-, Ethyl-, Propyl-, iso-Propyl-, n-Butyl-, iso-Butyl- und die tert.-Butylgruppe, vorzugsweise die Methyl- oder die Ethylgruppe und als
- Halogenalkylgruppen die Trifluormethyl-, Difluormethyl-, Fluormethyl-, Trichlormethyl-, Dichlormethyl-, Chlormethyl-, Difluorchlormethyl-, 1-Fluorethyl-, 2-Fluorethyl- und die 2,2,2-Trifluorethylgruppe, wobei die Trifluormethyl- und die Difluormethylgruppen bevorzugt sind,

in Betracht.

In der Bedeutung $R^2$ kommen als

- Alkylgruppen die unter $R^1$ genannten Reste, insbesondere die n-Propyl-, n-Butyl- sowie die iso-Butylgruppen,
- Alkenylgruppen z.B. Vinyl, Allyl, 2-Butenyl, 1-Methyl-2-propenyl, 2-Methyl-2-Propenyl, 2-Pentyl, 1-Methyl-2-pentenyl, 2,4-Pentadienyl und 1-Methyl-2,4-pentadienyl, bevorzugt der Allylrest, und als
- Alkinylgruppen z.B. 2-Propinyl, 1-Methyl-2-propinyl, 2-Methyl-2-propinyl sowie die isomeren Pentinyl- und Hexinylreste, wobei der 2-Propinylrest bevorzugt ist,

in Betracht.

In der Bedeutung B kommen als

- Alkylgruppen die unter $R^1$ genannten Reste sowie n-Pentyl, 2-Methylbutyl, 3-Methylbutyl, 2,2-Dimethylpropyl, n-Hexyl, 2-Methylpentyl, 3-Methylpentyl, 4-Methylpentyl, 2,2-Dimethylbutyl, 3,3-Dimethylbutyl, 2,3-Dimethylbutyl sowie 2-Ethylbutyl, insbesondere die n-Pentyl-, 3-Methylbutyl-, n-Hexyl- und die 4-Methylpentylgruppe;
- Alkenylgruppen die Vinylgruppe und die unter $R^2$ genannten Reste, wobei 2-Methyl-2-propenyl, 2-Pentenyl sowie 2,4-Pentadienyl bevorzugt sind;
- Alkinylgruppen die Ethinylgruppe und die unter $R^2$ genannten Reste, wobei 3-Butinyl, 3-Pentinyl sowie 4-Hexinyl bevorzugt sind;
- Cycloalkylgruppen z. B. Cyclopropyl, Cyclobutyl, Cyclopentyl, Cyclohexyl, Cycloheptyl sowie Adamantyl als Beispiel für ein überbrücktes System, wobei Cyclopentyl und Cyclohexyl bevorzugt sind;
- Cycloalkenylgruppen z. B. Cyclopropenyl, Cyclobutenyl, Cyclopentenyl, Cyclohexenyl und Cycloheptenyl sowie Norbornenyl als Beispiel für ein überbrücktes System, bevorzugt Cyclopentenyl, Cyclohexenyl und Norbornenyl;
- heterocyclische Aliphaten z.B. Epoxyethyl, Oxetanyl, Tetrahydrofuranyl, Tetrahydropyranyl, Thietanyl, Tetrahydrothienyl oder Tetrahydrothiopyranyl sowie Xanthenyl als Beispiel für eine benzokondensierte Gruppe, bevorzugt der Tetrahydrofuranyl-, Tetrahydropyranyl-, Tetrahydrothienyl-, Tetrahydrothiopyranyl- sowie der Xanthenylrest;
- heterocyclische Alkenylringe u.a. der Dihydrofuranyl-, Dihydrothienyl- und Dihydrothiopyranylrest, insbesondere jedoch der Dihydropyranyl- und der Dihydrothienylrest;
- isoaromatische Reste z. B. Phenyl, Naphthyl, Anthracenyl und Phenanthrenyl, besonders aber der Phenyl- und der Naphthylring, und als
- heteroaromatische Gruppen z. B. Pyrrolyl, Pyrazolyl, Imidazolyl, Triazolyl, Pyridyl, Pyrimidyl, Pyrazinyl, Pyridazinyl, Chinolinyl oder Isochinolinyl, Furanyl, Thienyl, Isoxazolyl, Oxazolyl, Oxathiazolyl, Isothiazolyl, Thioxalyl oder Thiodioxalyl ; bevorzugt sind Pyrrolyl, Pyrazolyl, Imidazolyl, Pyridyl, Chinolinyl, Furanyl, Thienyl, Isoxazolyl, Oxazolyl, Isothiazoyl sowie Thioxazolyl

in Betracht.

Bevorzugte Substituenten von B sind

- Alkylgruppen wie die unter $R^1$ genannten Reste, insbesondere Methyl, Ethyl und iso-Propyl;
- Alkenylgruppen wie die Vinylgruppe sowie die unter $R^2$ genannten Reste, bevorzugt Vinyl, 2-Propenyl und 2-Butenyl;
- Alkinylgruppen wie die Ethinylgruppe sowie die unter $R^2$ genannten Reste, besonders aber Ethinyl und 2-Propinyl;
- Halogenalkylgruppen wie die unter $R^1$ genannten Reste, bevorzugt die Trifluormethyl- und die Difluormethylgruppe;
- Halogenatome wie das Fluor-, Chlor-, Brom- oder Jodatom, vorzugsweise jedoch das Fluoratom und das Chloratom;
- Alkyloxygruppen wie die Methoxy-, Ethoxy-, n-Propyloxy-, iso-Propyloxy- sowie die isomeren Butoxyreste, insbesondere die Methoxy- und die Ethoxygruppe;

- Alkylthiogruppen wie die Methylthio-, Ethylthio-, n-Propylthio-, iso-Propylthio- und die isomeren Butylthioreste, besonders die Methylthio- und die Ethylthiogruppe.

Die 5-Amino-6-pyridazone Ia und/oder Ib bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia und/oder Ib eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen 5-Amino-6-pyridazone können beispielsweise wie folgt formuliert werden:

I. Man vermischt 90 Gewichtsteile der Verbindung Nr. 1.019 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.036 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.039 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.065 werden in einer Mischung gelöst, die aus 25 Gewichtsteilen Cyclohexanon, 65 Gewichtsteilen einer Mineralölfraktion vom Siedepunkt 210 bis 280 °C und 10

Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.094 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.139 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.119 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.443 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 1,0 kg/ha.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |

| Botanischer Name | Deutscher Name |
|---|---|
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Pyridazinone der Formel Ia und/oder Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone,

Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die neuen Verbindungen der Formel Ia und/oder Ib allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Die in den nachstehenden Synthesebeispielen wiedergegebenen Vorschriften wurden unter entsprechender Abwandlung der Ausgangsverbindungen zur Gewinnung weiterer Verbindungen der Formel Ia und/oder Ib benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Synthesebeispiele:

Beispiel 1

5-Acetylamino-1-methyl-6-pyridazon

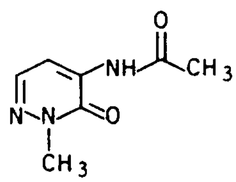

a) 1930 ml Hydrazinhydrat wurden bei 80 °C portionsweise mit 240,8 g (1,38 mol) 5-Chlor-4-methoxy-1-methyl-6-pyridazon so versetzt, daß die Temperatur ohne weiteres Erhitzen bei 80 °C blieb. Nach dem Abkühlen wurde der Niederschlag abfiltriert, gewaschen und getrocknet. Man erhielt so 112,8 g (65,3 %) 5-Amino-1-methyl-6-pyridazon vom Fp.: 191 bis 192 °C.

Das benötigte 5-Chlor-4-methoxy-1-methyl-6-pyridazon wurde nach DE-OS 25 26 643 wie folgt hergestellt:

295 g 30 %ige Natriummethylatlösung wurden so zu 293 g (1,64 mol) 4,5-Dichlor-1-methyl-6-pyridazon in 690 ml Methanol gegeben, daß die Temperatur 30 °C nicht überschritt. Nach dem Abkühlen und 12 h Rühren bei 25 °C wurde der gebildete Niederschlag abfiltriert, gewaschen und getrocknet. Man erhielt so 241 g (84,3 %) 5-Chlor-4-methoxy-1-methyl-6-pyridazon vom Fp. 189 bis 191 °C.

b) Eine Losung aus 4,0 g (0,032 mol) 5-Amino-1-methyl-6-pyridazon in 100 ml Pyridin wurde tropfenweise mit einer Lösung aus 2,7 ml Acetylchlorid in 20 ml Dioxan versetzt. Nach 12 h Rühren bei 25 °C wurden die Lösungsmittel unter vermindertem Druck abdestilliert, der Rückstand mit Methylenchlorid aufgenommen, mit Natriumhydrogencarbonat-Lösung gewaschen und getrocknet. Nach dem Abziehen des Lösungsmittels unter vermindertem Druck und Ausrühren des Rückstands mit wenig Diisopropylether erhielt man 3,1 g (58,0 %) 5-Acetylamino-1-methyl-6-pyridazon (Wirkstoffbeispiel 1.001) vom Fp: 152 bis 153 °C.

Beispiel 2

5-(2-Brombenzamido)-1-methyl-6-pyridazon

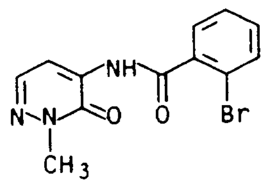

Aus 4,0 g (0,032 mol) 5-Amino-1-methyl-6-pyridazon in 100 ml Pyridin und 7,7 g (0,035 mol) 2-Brombenzoylchlorid in 20 ml Dioxan erhielt man analog Beispiel 1b) 8,0 g (81,2 %) 5-(2-Brombenzamido)-1-methyl-6-pyridazon (Wirkstoffbeispiel 1.082) vom Fp. 190 bis 193 ° C.

Beispiel 3

5-[N-(2-Brombenzoyl)-N-methyl]-amino-1-methyl-6-pyridazon

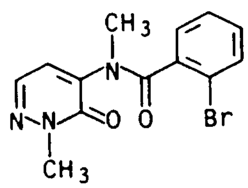

4,8 g (0,0156 mol) 5-(2-Brombenzamido)-1-methyl-6-pyridazon in 50 ml Dimethylformamid wurden mit 2,9 g 30 %iger Natriummethylatlösung versetzt. Anschließend wurde das Methanol vollständig unter vermindertem Druck abgezogen und die Reaktionsmischung mit 3 g Dimethylsulfat 2,5 h bei 80 ° C gerührt. Zur Aufarbeitung wurde mit Wasser und 20 ml konz. Ammoniaklösung versetzt und das Produkt mit Essigester extrahiert. Nach Waschen und Trocknen wurde die organische Phase eingeengt. Säulenchromatographie an Kieselgel (Eluent: Cyclohexan/Essigester) lieferte 3,8 g (75,5 %) 5-[N-(2-Brombenzoyl)-N-methyl]-amino-1-methyl-6-pyridazon (Wirkstoffbeispiel 2.382) als zähes Öl.
$^1$H-NMR (DMSO-$d_6$): 3,2 (s, 3H), 3,6 (s, 3H), 7,3 (m, 4H), 7,5 (m, 1H), 7,8 (d, J = 5 Hz, 1H) ppm.

Tabelle 1

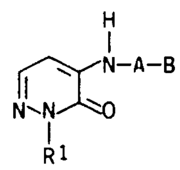

| Nr. | B | A | $R^1$ | Physik. Daten Fp (°C) $^1H$-NMR (ppm) |
|---|---|---|---|---|
| 1.001 | Methyl | CO | $CH_3$ | 152-153 |
| 1.002 | Methyl | $SO_2$ | $CH_3$ | |
| 1.003 | Ethyl | CO | $CH_3$ | 115-117 |
| 1.004 | Ethyl | $SO_2$ | $CH_3$ | |
| 1.005 | Isopropyl | CO | $CH_3$ | |
| 1.006 | tert.-Butyl | CO | $CH_3$ | |
| 1.007 | tert.-Bulylmethyl | CO | $CH_3$ | |
| 1.008 | Chlormethyl | CO | $CH_3$ | |
| 1.009 | Dichlormethyl | CO | $CH_3$ | |
| 1.010 | 2-Chlorethyl | CO | $CH_3$ | |
| 1.011 | Methoxymethyl | CO | $CH_3$ | |
| 1.012 | Ethenyl | CO | $CH_3$ | |
| 1.013 | 1-Methylethenyl | CO | $CH_3$ | |
| 1.014 | Ethinyl | CO | $CH_3$ | |
| 1.015 | Benzyl | CO | $CH_3$ | 154-156 |
| 1.016 | Cinnamyl | CO | $CH_3$ | 155-157 |
| 1.017 | Cyclopropyl | CO | $CH_3$ | 165-166 |
| 1.018 | 1-Methylcyclopropyl | CO | $CH_3$ | |
| 1.019 | 2-Methylcyclopropyl | CO | $CH_3$ | 138-140 |
| 1.020 | 1-Phenylcyclopropyl | CO | $CH_3$ | |
| 1.021 | 2,2-Dichlor-1-methyl-cyclopropyl | CO | $CH_3$ | |
| 1.022 | Cyclopentyl | CO | $CH_3$ | |
| 1.023 | 1-Phenylcyclopentyl | CO | $CH_3$ | |
| 1.024 | 9-Fluorenyl | CO | $CH_3$ | |
| 1.025 | Cyclohexyl | CO | $CH_3$ | 138-139 |
| 1.026 | 1-Methylcyclohexyl | CO | $CH_3$ | 95-97 |
| 1.027 | 2-Methyl-norborn-2-en-5-yl | CO | $CH_3$ | 104-150 |
| 1.028 | Tetrahydropyran-3-yl | CO | $CH_3$ | |
| 1.029 | Tetrahydropyran-4-yl | CO | $CH_3$ | |
| 1.030 | 5,6-Dihydro-2-methyl-4H-pyran-3-yl | CO | $CH_3$ | 129-130 |

Tabelle 1 - Forts.

| Nr. | B | A | $R^1$ | Physik. Daten Fp (°C) $^1$H-NMR (ppm) |
|---|---|---|---|---|
| 1.031 | 5,6-Dihydro-2,6,6-trimethyl-4H-pyran-3-yl | CO | $CH_3$ | |
| 1.032 | Xanthen-9-yl | CO | $CH_3$ | |
| 1.033 | 5,6-Dihydro-3-methyl-4H-thiopyran-2-yl | CO | $CH_3$ | |
| 1.034 | Tetrahydrothiopyran-3-yl | CO | $CH_3$ | 156-157 |
| 1.035 | Tetrahydrothiopyran-4-yl | CO | $CH_3$ | |
| 1.036 | Phenyl | CO | $CH_3$ | 111-112 |
| 1.037 | Phenyl | CO | $CH(CH_3)_2$ | |
| 1.038 | Phenyl | CO | $C(CH_3)_3$ | |
| 1.039 | Phenyl | $SO_2$ | $CH_3$ | 161-163 |
| 1.040 | 2-Methylphenyl | CO | $CH_3$ | 144-146 |
| 1.041 | 2-Methylphenyl | $SO_2$ | $CH_3$ | |
| 1.042 | 2-Methylphenyl | $SO_2$ | $CH_3$ | |
| 1.043 | 4-Methylphenyl | CO | $CH_3$ | 151-153 |
| 1.044 | 2,4-Dimethylphenyl | CO | $CH_3$ | |
| 1.045 | 2,4-Dimethylphenyl | $SO_2$ | $CH_3$ | |
| 1.046 | 2,6-Dimethylphenyl | CO | $CH_3$ | |
| 1.047 | 2,6-Dimethylphenyl | $SO_2$ | $CH_3$ | |
| 1.048 | 2-Trifluormethylphenyl | CO | $CH_3$ | 185-187 |
| 1.049 | 2-Trifluormethylphenyl | $SO_2$ | $CH_3$ | |
| 1.050 | 2-Methoxyphenyl | CO | $CH_3$ | 160-163 |
| 1.051 | 2-Methoxyphenyl | $SO_2$ | $CH_3$ | |
| 1.052 | 2,6-Dimethoxyphenyl | CO | $CH_3$ | 160-164 |
| 1.053 | 2,6-Dimethoxyphenyl | $SO_2$ | $CH_3$ | |
| 1.054 | 2-Trifluormethoxyphenyl | CO | $CH_3$ | |
| 1.055 | 2-Methylthiophenyl | CO | $CH_3$ | |
| 1.056 | 2-Methylsulfinylphenyl | CO | $CH_3$ | |
| 1.057 | 2-Methylsulfonylphenyl | CO | $CH_3$ | |
| 1.058 | 2-Fluorphenyl | CO | $CH_3$ | 194-200 |
| 1.059 | 2-Fluorphenyl | $SO_2$ | $CH_3$ | |
| 1.060 | 2-Fluorphenyl | CO | $CH_2-CF_3$ | |
| 1.061 | 2,3-Difluorphenyl | CO | $CH_3$ | |
| 1.062 | 2,4-Difluorphenyl | CO | $CH_3$ | 198-200 |
| 1.063 | 2,5-Difluorphenyl | CO | $CH_3$ | |
| 1.064 | 2,6-Difluorphenyl | CO | $CH_3$ | 198-206 |
| 1.065 | 2-Chlorphenyl | CO | $CH_3$ | 176-178 |
| 1.066 | 2-Chlorphenyl | $SO_2$ | $CH_3$ | 129-136 |
| 1.067 | 2-Chlorphenyl | CO | $CH(CH_3)_2$ | 95-97 |

Tabelle 1 - Forts.

| Nr. | B | A | $R^1$ | Physik. Daten Fp (°C) $^1H$-NMR (ppm) |
|---|---|---|---|---|
| 1.068 | 2-Chlorphenyl | CO | $C(CH_3)_3$ | 84-86 |
| 1.069 | 2-Chlorphenyl | CO | $CH_2CF_3$ | |
| 1.070 | 3-Chlorphenyl | CO | $CH_3$ | 151-153 |
| 1.071 | 3-Chlorphenyl | $SO_2$ | $CH_3$ | |
| 1.072 | 4-Chlorphenyl | CO | $CH_3$ | 231-232 |
| 1.073 | 4-Chlorphenyl | $SO_2$ | $CH_3$ | |
| 1.074 | 2,3-Dichlorphenyl | CO | $CH_3$ | |
| 1.075 | 2,4-Dichlorphenyl | CO | $CH_3$ | 153-154 |
| 1.076 | 2,5-Dichlorphenyl | CO | $CH_3$ | 164-166 |
| 1.077 | 2,6-Dichlorphenyl | CO | $CH_3$ | |
| 1.078 | 2,6-Dichlorphenyl | $SO_2$ | $CH_3$ | |
| 1.079 | 2,4,6-Trichlorphenyl | CO | $CH_3$ | |
| 1.080 | 2-Chlor-6-methylphenyl | CO | $CH_3$ | |
| 1.081 | 2-Chlor-6-methylphenyl | $SO_2$ | $CH_3$ | |
| 1.082 | 2-Bromphenyl | CO | $CH_3$ | 190-193 |
| 1.083 | 2-Bromphenyl | $SO_2$ | $CH_3$ | |
| 1.084 | 2-Cyanophenyl | CO | $CH_3$ | |
| 1.085 | 2-Cyanophenyl | $SO_2$ | $CH_3$ | |
| 1.086 | 2-Nitrophenyl | CO | $CH_3$ | 178-186 |
| 1.087 | 2-Nitrophenyl | $SO_2$ | $CH_3$ | |
| 1.088 | 2-Carbomethoxyphenyl | CO | $CH_3$ | |
| 1.089 | 2-Carbomethoxyphenyl | $SO_2$ | $CH_3$ | |
| 1.090 | 2-Carbethoxyphenyl | CO | $CH_3$ | |
| 1.091 | 2-Carbethoxyphenyl | $SO_2$ | $CH_3$ | |
| 1.092 | 2-(N,N-Dimethylcarbamido)phenyl | CO | $CH_3$ | |
| 1.093 | 2-Pyridyl | CO | $CH_3$ | |
| 1.094 | 3-Pyridyl | CO | $CH_3$ | 136-137 |
| 1.095 | 2-Fluor-3-pyridyl | CO | $CH_3$ | 154-155 |
| 1.096 | 2-Chlor-3-pyridyl | CO | $CH_3$ | |
| 1.097 | 4-Chlor-3-pyridyl | CO | $CH_3$ | |
| 1.098 | 4-Pyridyl | CO | $CH_3$ | |
| 1.099 | 5-Pyrimidyl | CO | $CH_3$ | |
| 1.100 | 1-Naphthyl | CO | $CH_3$ | |
| 1.101 | 2-Naphthyl | CO | $CH_3$ | |
| 1.102 | 2-Chinolinyl | CO | $CH_3$ | |
| 1.103 | 3-Chinolinyl | CO | $CH_3$ | |
| 1.104 | 2-Methyl-4-chinolinyl | CO | $CH_3$ | |
| 1.105 | 3,7-Dichlor-8-chinolinyl | CO | $CH_3$ | |

Tabelle 1 - Forts.

| Nr. | B | A | $R^1$ | Physik. Daten Fp (°C) $^1$H-NMR (ppm) |
|---|---|---|---|---|
| 1.106 | 7-Chlor-3-methyl-8-chinolinyl | CO | $CH_3$ | |
| 1.107 | 7-Chlor-3-methyl-8-chinolinyl | CO | $CH_3$ | |
| 1.108 | 2-Pyrolyl | CO | $CH_3$ | |
| 1.109 | 3-Pyrolyl | CO | $CH_3$ | |
| 1.110 | 3-Chlor-2-pyrolyl | CO | $CH_3$ | |
| 1.111 | 1-Methyl-2-pyrolyl | CO | $CH_3$ | 137-133 |
| 1.112 | 2-Furanyl | CO | $CH_3$ | |
| 1.113 | 3-Methyl-2-furanyl | CO | $CH_3$ | |
| 1.114 | 5-Brom-2-furanyl | CO | $CH_3$ | 165-166 |
| 1.115 | 5-Methyl-2-furanyl | CO | $CH_3$ | |
| 1.116 | 3-Furanyl | CO | $CH_3$ | |
| 1.117 | 2,5-Dimethyl-3-furanyl | CO | $CH_3$ | 123-125 |
| 1.118 | 2,4,5-Trimethyl-3-furanyl | CO | $CH_3$ | |
| 1.119 | 2-Thienyl | CO | $CH_3$ | 162-164 |
| 1.120 | 3-Chlor-2-thienyl | CO | $CH_3$ | 224-226 |
| 1.121 | 5-Methyl-2-thienyl | CO | $CH_3$ | 147-149 |
| 1.122 | 3-Thienyl | CO | $CH_3$ | |
| 1.123 | 4-Chlor-3-thienyl | CO | $CH_3$ | 165-167 |
| 1.124 | 1-Methyl-5-pyrazolyl | CO | $CH_3$ | |
| 1.125 | 4-Pyrazolyl | CO | $CH_3$ | |
| 1.126 | 1-Methyl-4-pyrazolyl | CO | $CH_3$ | |
| 1.127 | 3,5-Dimethyl-4-pyrazolyl | CO | $CH_3$ | |
| 1.128 | 1-Methyl-2-imidazolyl | CO | $CH_3$ | |
| 1.129 | 4-Imidazolyl | CO | $CH_3$ | |
| 1.130 | 2-Methyl-4-imidazolyl | CO | $CH_3$ | |
| 1.131 | 4-Methyl-5-imidazolyl | CO | $CH_3$ | |
| 1.132 | 5-Isoxazolyl | CO | $CH_3$ | |
| 1.133 | 4-Isoxazolyl | CO | $CH_3$ | |
| 1.134 | 3-Isopropyl-5-isoxazolyl | CO | $CH_3$ | 104-105 |
| 1.135 | 3-Methyl-4-isoxazolyl | CO | $CH_3$ | |
| 1.136 | 4-Isothiazolyl | CO | $CH_3$ | |
| 1.137 | 1-Methyl-4-carbethoxy--3-pyrazolyl | CO | $CH_3$ | 194-196 |
| 1.138 | 5-Chlor-2-thienyl | CO | $CH_3$ | 158-160 |
| 1.139 | 2-Thienyl | $SO_2$ | $CH_3$ | 186-188 |
| 1.140 | 2,5-Dichlor-3-thienyl | $SO_2$ | $CH_3$ | 152-154 |

Tabelle 1 - Forts.

| Nr. | B | A | $R^1$ | Physik. Daten Fp (°C) $^1H$-NMR (ppm) |
|---|---|---|---|---|
| 1.141 | 2,5-Dichlor-3-thienyl | CO | $CH_3$ | 170-171 |
| 1.142 | 3-Chlor-2-thienyl | $SO_2$ | $CH_3$ | 185-187 |
| 1.143 | 2-Chlor-3-thienyl | CO | $CH_3$ | 185-186 |
| 1.144 | 1-Adamantyl | CO | $CH_3$ | 173 |
| 1.145 | Cyclobuyl | CO | $CH_3$ | 139-141 |
| 1.146 | 4-Methyl-5-oxazolyl | CO | $CH_3$ | |
| 1.147 | 2-Methyl-4-oxazolyl | CO | $CH_3$ | |
| 1.148 | 3-Isopropyl-4-isoxazolyl | CO | $CH_3$ | |

Tabelle 2

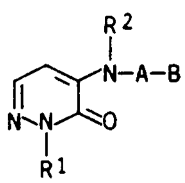

| Nr. | B | A | $R^1$ | $R^2$ | Physik. Daten Fp (°C) $^1H$-NMR (ppm) |
|---|---|---|---|---|---|
| 2.001 | Methyl | CO | $CH_3$ | $CH_3$ | |
| 2.002 | Methyl | CO | $CH_3$ | $CH_2CH_2$ | |
| 2.003 | Methyl | CO | $CH_3$ | $CH(CH_3)_2$ | |
| 2.004 | Methyl | CO | $CH_3$ | $CH_2CH=CH_2$ | |
| 2.005 | Methyl | CO | $CH_3$ | $CH_2C\equiv CH$ | |
| 2.006 | Methyl | CO | $CH_3$ | $CH_2Ph$ | |
| 2.007 | Methyl | $SO_2$ | $CH_3$ | $CH_3$ | |
| 2.008 | Methyl | $SO_2$ | $CH_3$ | $CH_2CH_3$ | |
| 2.009 | Methyl | $SO_2$ | $CH_3$ | $CH(CH_3)_2$ | |
| 2.010 | Methyl | $SO_2$ | $CH_3$ | $CH_2CH=CH_2$ | |
| 2.011 | Methyl | $SO_2$ | $CH_3$ | $CH_2C\equiv CH$ | |
| 2.012 | Methyl | $SO_2$ | $CH_3$ | $CH_2Ph$ | |
| 2.013 | Ethyl | CO | $CH_3$ | $CH_3$ | |
| 2.014 | Ethyl | CO | $CH_3$ | $CH_2CH_3$ | |

Tabelle 2 - Forts.

| Nr. | B | | A | $R^1$ | Physik. Daten Fp (°C) $^1$H-NMR (ppm) |
|---|---|---|---|---|---|
| 2.015 | Ethyl | CO | $CH_3$ | $CH(CH_3)_2$ | |
| 2.016 | Ethyl | CO | $CH_3$ | $CH_2CH=CH_2$ | |
| 2.017 | Ethyl | CO | $CH_3$ | $CH_2C\equiv CH$ | |
| 2.018 | Ethyl | CO | $CH_3$ | $CH_2Ph$ | |
| 2.019 | Ethyl | $SO_2$ | $CH_3$ | $CH_3$ | |
| 2.020 | Ethyl | $SO_2$ | $CH_3$ | $CH_2CH_3$ | |
| 2.021 | Ethyl | $SO_2$ | $CH_3$ | $CH(CH_3)_2$ | |
| 2.022 | Ethyl | $SO_2$ | $CH_3$ | $CH_2CH=CH_2$ | |
| 2.023 | Ethyl | $SO_2$ | $CH_3$ | $CH_2C\equiv CH$ | |
| 2.024 | Ethyl | $SO_2$ | $CH_3$ | $CH_2Ph$ | |
| 2.025 | Isopropyl | CO | $CH_3$ | $CH_3$ | |
| 2.026 | Isopropyl | CO | $CH_3$ | $CH_2CH_3$ | |
| 2.027 | Isopropyl | CO | $CH_3$ | $CH(CH_3)_2$ | |
| 2.028 | Isopropyl | CO | $CH_3$ | $CH_2CH=CH_2$ | |
| 2.029 | Isopropyl | CO | $CH_3$ | $CH_2C\equiv CH$ | |
| 2.030 | Isopropyl | CO | $CH_3$ | $CH_2Ph$ | |
| 2.031 | tert.-Butyl | CO | $CH_3$ | $CH_3$ | |
| 2.032 | tert.-Butyl | CO | $CH_3$ | $CH_2CH_3$ | |
| 2.033 | tert.-Butyl | CO | $CH_3$ | $CH(CH_3)_2$ | |
| 2.034 | tert.-Butyl | CO | $CH_3$ | $CH_2CH=CH_2$ | |
| 2.035 | tert.-Butyl | CO | $CH_3$ | $CH_2C\equiv CH$ | |
| 2.036 | tert.-Butyl | CO | $CH_3$ | $CH_2Ph$ | |
| 2.037 | tert.-Butylmethyl | CO | $CH_3$ | $CH_3$ | |
| 2.038 | tert.-Butylmethyl | CO | $CH_3$ | $CH_2CH_3$ | |
| 2.039 | tert.-Butylmethyl | CO | $CH_3$ | $CH(CH_3)_2$ | |
| 2.040 | tert.-Butylmethyl | CO | $CH_3$ | $CH_2CH=CH_2$ | |
| 2.041 | tert.-Butylmethyl | CO | $CH_3$ | $CH_2C\equiv CH$ | |
| 2.042 | tert.-Butylmethyl | CO | $CH_3$ | $CH_2Ph$ | |
| 2.043 | Chlormethyl | CO | $CH_3$ | $CH_3$ | |
| 2.044 | Chlormethyl | CO | $CH_3$ | $CH_2CH_3$ | |
| 2.045 | Chlormethyl | CO | $CH_3$ | $CH(CH_3)_2$ | |
| 2.046 | Chlormethyl | CO | $CH_3$ | $CH_2CH=CH_2$ | |
| 2.047 | Chlormethyl | CO | $CH_3$ | $CH_2C\equiv CH$ | |
| 2.048 | Chlormethyl | CO | $CH_3$ | $CH_2Ph$ | |
| 2.049 | Dichlormethyl | CO | $CH_3$ | $CH_3$ | |
| 2.050 | Dichlormethyl | CO | $CH_3$ | $CH_2CH_3$ | |
| 2.051 | Dichlormethyl | CO | $CH_3$ | $CH(CH_3)_2$ | |

Tabelle 2 - Forts.

| Nr. | B | | A | $R^1$ | Physik. Daten Fp (°C) $^1$H-NMR (ppm) |
|---|---|---|---|---|---|
| 2.052 | Dichlormethyl | CO | $CH_3$ | $CH_2CH=CH_2$ | |
| 2.053 | Dichlormethyl | CO | $CH_3$ | $CH_2C\equiv CH$ | |
| 2.054 | Dichlormethyl | CO | $CH_3$ | $CH_2Ph$ | |
| 2.055 | 2-Chlorethyl | CO | $CH_3$ | $CH_3$ | |
| 2.056 | 2-Chlorethyl | CO | $CH_3$ | $CH_2CH_3$ | |
| 2.057 | 2-Chorethyl | CO | $CH_3$ | $CH(CH_3)_2$ | |
| 2.058 | 2-Chlorethyl | CO | $CH_3$ | $CH_2CH=CH_2$ | |
| 2.059 | 2-Chlorethyl | CO | $CH_3$ | $CH_2C\equiv CH$ | |
| 2.060 | 2-Chlorethyl | CO | $CH_3$ | $CH_2Ph$ | |
| 2.061 | Methoxymethyl | CO | $CH_3$ | $CH_3$ | |
| 2.062 | Methoxymethyl | CO | $CH_3$ | $CH_2CH_3$ | |
| 2.063 | Methoxymethyl | CO | $CH_3$ | $CH(CH_3)_2$ | |
| 2.064 | Methoxymethyl | CO | $CH_3$ | $CH_2CH=CH_2$ | |
| 2.065 | Methoxymethyl | CO | $CH_3$ | $CH_2C\equiv CH$ | |
| 2.066 | Methoxymethyl | CO | $CH_3$ | $CH_2Ph$ | |
| 2.067 | Ethenyl | CO | $CH_3$ | $CH_3$ | |
| 2.068 | 1-Methylethenyl | CO | $CH_3$ | $CH_3$ | |
| 2.069 | Ethinyl | CO | $CH_3$ | $CH_3$ | |
| 2.070 | Benzyl | CO | $CH_3$ | $CH_3$ | |
| 2.071 | Cinnamyl | CO | $CH_3$ | $CH_3$ | |
| 2.072 | Cyclopropyl | CO | $CH_3$ | $CH_3$ | |
| 2.073 | Cyclopropyl | CO | $CH_3$ | $CH_2CH_3$ | |
| 2.074 | Cyclopropyl | CO | $CH_3$ | $CH(CH_3)_2$ | |
| 2.075 | Cyclopropyl | CO | $CH_3$ | $CH_2CH=CH_2$ | |
| 2.076 | Cyclopropyl | CO | $CH_3$ | $CH_2C\equiv CH$ | |
| 2.077 | Cyclopropyl | CO | $CH_3$ | $CH_2Ph$ | |
| 2.078 | 1-Methylcyclopropyl | CO | $CH_3$ | $CH_3$ | |
| 2.079 | 1-Methylcyclopropyl | CO | $CH_3$ | $CH_2CH_3$ | |
| 2.080 | 1-Methylcyclopropyl | CO | $CH_3$ | $CH(CH_3)_2$ | |
| 2.081 | 1-Methylcyclopropyl | CO | $CH_3$ | $CH_2CH=CH_2$ | |
| 2.082 | 1-Methylcyclopropyl | CO | $CH_3$ | $CH_2C\equiv CH$ | |
| 2.083 | 1-Methylcyclopropyl | CO | $CH_3$ | $CH_2Ph$ | |
| 2.084 | 2-Methylcyclopropyl | CO | $CH_3$ | $CH_3$ | |
| 2.085 | 2-Methylcyclopropyl | CO | $CH_3$ | $CH_2CH_3$ | |
| 2.086 | 2-Methylcyclopropyl | CO | $CH_3$ | $CH(CH_3)_2$ | |
| 2.087 | 2-Methylcyclopropyl | CO | $CH_3$ | $CH_2CH=CH_2$ | |
| 2.088 | 2-Methylcyclopropyl | CO | $CH_3$ | $CH_2C\equiv CH$ | |
| 2.089 | 2-Methylcyclopropyl | CO | $CH_3$ | $CH_2Ph$ | |

Tabelle 2 - Forts.

| Nr. | B | | A | $R^1$ | Physik. Daten Fp (°C) $^1H$-NMR (ppm) |
|---|---|---|---|---|---|
| 2.090 | 1-Phenylcyclopropyl | CO | $CH_3$ | $CH_3$ | |
| 2.091 | 1-Phenylcyclopropyl | CO | $CH_3$ | $CH_2CH_3$ | |
| 2.092 | 1-Phenylcyclopropyl | CO | $CH_3$ | $CH(CH_3)_2$ | |
| 2.093 | 1-Phenylcyclopropyl | CO | $CH_3$ | $CH_2CH{=}CH_2$ | |
| 2.094 | 1-Phenylcyclopropyl | CO | $CH_3$ | $CH_2C{\equiv}CH$ | |
| 2.095 | 1-Phenylcyclopropyl | CO | $CH_3$ | $CH_2Ph$ | |
| 2.096 | 2,2-Dichlor-1-methyl-cyclopropyl | CO | $CH_3$ | $CH_3$ | |
| 2.097 | 2,2-Dichlor-1-methyl-cyclopropyl | CO | $CH_3$ | $CH_2CH_3$ | |
| 2.098 | 2,2-Dichlor-1-methyl-cyclopropyl | CO | $CH_3$ | $CH(CH_3)_2$ | |
| 2.099 | 2,2-Dichlor-1-methyl-cyclopropyl | CO | $CH_3$ | $CH_2CH{=}CH_2$ | |
| 2.100 | 2,2-Dichlor-1-methyl-cyclopropyl | CO | $CH_3$ | $CH_2C{\equiv}CH$ | |
| 2.101 | 2,2-Dichlor-1-methyl-cyclopropyl | CO | $CH_3$ | $CH_2Ph$ | |
| 2.102 | Cyclopentyl | CO | $CH_3$ | $CH_3$ | |
| 2.103 | Cyclopentyl | CO | $CH_3$ | $CH_2CH_3$ | |
| 2.104 | Cyclopentyl | CO | $CH_3$ | $CH(CH_3)_2$ | |
| 2.105 | Cyclopentyl | CO | $CH_3$ | $CH_2CH{=}CH_2$ | |
| 2.106 | Cyclopentyl | CO | $CH_3$ | $CH_2C{\equiv}CH$ | |
| 2.107 | Cyclopentyl | CO | $CH_3$ | $CH_2Ph$ | |
| 2.108 | 1-Phenylcyclopentyl | CO | $CH_3$ | $CH_3$ | |
| 2.109 | 1-Phenylcyclopentyl | CO | $CH_3$ | $CH_2CH_3$ | |
| 2.110 | 1-Phenylcyclopentyl | CO | $CH_3$ | $CH(CH_3)_2$ | |
| 2.111 | 1-Phenylcyclopentyl | CO | $CH_3$ | $CH_2CH{=}CH_2$ | |
| 2.112 | 1-Phenylcyclopentyl | CO | $CH_3$ | $CH_2C{\equiv}CH$ | |
| 2.113 | 1-Phenylcyclopentyl | CO | $CH_3$ | $CH_2Ph$ | |
| 2.114 | 9-Fluorenyl | CO | $CH_3$ | $CH_3$ | |
| 2.115 | 9-Fluorenyl | CO | $CH_3$ | $CH_2CH_3$ | |
| 2.116 | 9-Fluorenyl | CO | $CH_3$ | $CH(CH_3)_2$ | |
| 2.117 | 9-Fluorenyl | CO | $CH_3$ | $CH_2CH{=}CH_2$ | |
| 2.118 | 9-Fluorenyl | CO | $CH_3$ | $CH_2C{\equiv}CH$ | |
| 2.119 | 9-Fluorenyl | CO | $CH_3$ | $CH_2Ph$ | |
| 2.120 | Cyclohexyl | CO | $CH_3$ | $CH_3$ | |

Tabelle 2 - Forts.

| Nr. | B | | A | $R^1$ | Physik. Daten Fp (°C) $^1$H-NMR (ppm) |
|---|---|---|---|---|---|
| 2.121 | Cyclohexyl | CO | $CH_3$ | $CH_2CH_3$ | |
| 2.122 | Cyclohexyl | CO | $CH_3$ | $CH(CH_3)_2$ | |
| 2.123 | Cyclohexyl | CO | $CH_3$ | $CH_2CH=CH_2$ | |
| 2.124 | Cyclohexyl | CO | $CH_3$ | $CH_2C\equiv CH$ | |
| 2.125 | Cyclohexyl | CO | $CH_3$ | $CH_2Ph$ | |
| 2.126 | 1-Methylcyclohexyl | CO | $CH_3$ | $CH_3$ | |
| 2.127 | 1-Methylcyclohexyl | CO | $CH_3$ | $CH_2CH_3$ | |
| 2.128 | 1-Methylcyclohexyl | CO | $CH_3$ | $CH(CH_3)_2$ | |
| 2.129 | 1-Methylcyclohexyl | CO | $CH_3$ | $CH_2CH=CH_2$ | |
| 2.130 | 1-Methylcyclohexyl | CO | $CH_3$ | $CH_2C\equiv CH$ | |
| 2.131 | 1-Methylcyclohexyl | CO | $CH_3$ | $CH_2Ph$ | |
| 2.132 | 5-Norbornen-2-yl | CO | $CH_3$ | $CH_3$ | |
| 2.133 | 5-Norbornen-2-yl | CO | $CH_3$ | $CH_2CH_3$ | |
| 2.134 | 5-Norbornen-2-yl | CO | $CH_3$ | $CH(CH_3)_2$ | |
| 2.135 | 5-Norbornen-2-yl | CO | $CH_3$ | $CH_2CH=CH_2$ | |
| 2.136 | 5-Norbornen-2-yl | CO | $CH_3$ | $CH_2C\equiv CH$ | |
| 2.137 | 5-Norbornen-2-yl | CO | $CH_3$ | $CH_2Ph$ | |
| 2.138 | Tetrahydropyran-3-yl | CO | $CH_3$ | $CH_3$ | |
| 2.139 | Tetrahydropyran-3-yl | CO | $CH_3$ | $CH_2CH_3$ | |
| 2.140 | Tetrahydropyran-3-yl | CO | $CH_3$ | $CH(CH_3)_2$ | |
| 2.141 | Tetrahydropyran-3-yl | CO | $CH_3$ | $CH_2CH=CH_2$ | |
| 2.142 | Tetrahydropyran-3-yl | CO | $CH_3$ | $CH_2C\equiv CH$ | |
| 2.143 | Tetrahydropyran-3-yl | CO | $CH_3$ | $CH_2Ph$ | |
| 2.144 | Tetrahydropyran-4-yl | CO | $CH_3$ | $CH_3$ | |
| 2.145 | Tetrahydropyran-4-yl | CO | $CH_3$ | $CH_2CH_3$ | |
| 2.146 | Tetrahydropyran-4-yl | CO | $CH_3$ | $CH(CH_3)_2$ | |
| 2.147 | Tetrahydropyran-4-yl | CO | $CH_3$ | $CH_2CH=CH_2$ | |
| 2.148 | Tetrahydropyran-4-yl | CO | $CH_3$ | $CH_2C\equiv CH$ | |
| 2.149 | Tetrahydropyran-4-yl | CO | $CH_3$ | $CH_2Ph$ | |
| 2.150 | 5,6-Dihydro-2-methyl-4H-pyran-3-yl | CO | $CH_3$ | $CH_3$ | |
| 2.151 | 5,6-Dihydro-2-methyl-4H-pyran-3-yl | CO | $CH_3$ | $CH_2CH_3$ | |
| 2.152 | 5,6-Dihydro-2-methyl-4H-pyran-3-yl | CO | $CH_3$ | $CH(CH_3)_2$ | |
| 2.153 | 5,6-Dihydro-2-methyl-4H-pyran-3-yl | CO | $CH_3$ | $CH_2CH=CH_2$ | |

Tabelle 2 - Forts.

| Nr. | B | | A | $R^1$ | Physik. Daten Fp (°C) $^1$H-NMR (ppm) |
|---|---|---|---|---|---|
| 2.154 | 5,6-Dihydro-2-methyl-4H-pyran-3-yl | CO | $CH_3$ | $CH_2C{\equiv}CH$ | |
| 2.155 | 5,6-Dihydro-2-methyl-4H-pyran-3-yl | CO | $CH_3$ | $CH_2Ph$ | |
| 2.156 | 5,6-Dihydro-2,6,6-trimethyl-4H-pyran-3-yl | CO | $CH_3$ | $CH_3$ | |
| 2.157 | 5,6-Dihydro-2,6,6-trimethyl-4H-pyran-3-yl | CO | $CH_3$ | $CH_2CH_3$ | |
| 2.158 | 5,6-Dihydro-2,6,6-trimethyl-4H-pyran-3-yl | CO | $CH_3$ | $CH(CH_3)_2$ | |
| 2.159 | 5,6-Dihydro-2,6,6-trimethyl-4H-pyran-3-yl | CO | $CH_3$ | $CH_2CH{=}CH_2$ | |
| 2.160 | 5,6-Dihydro-2,6,6-trimethyl-4H-pyran-3-yl | CO | $CH_3$ | $CH_2C{\equiv}CH$ | |
| 2.161 | 5,6-Dihydro-2,6,6-trimethyl-4H-pyran-3-yl | CO | $CH_3$ | $CH_2Ph$ | |
| 2.162 | Xanthen-9-yl | CO | $CH_3$ | $CH_3$ | |
| 2.163 | Xanthen-9-yl | CO | $CH_3$ | $CH_2CH_3$ | |
| 2.164 | Xanthen-9-yl | CO | $CH_3$ | $CH(CH_3)_2$ | |
| 2.165 | Xanthen-9-yl | CO | $CH_3$ | $CH_2CH{=}CH_2$ | |
| 2.166 | Xanthen-9-yl | CO | $CH_3$ | $CH_2C{\equiv}CH$ | |
| 2.167 | Xanthen-9-yl | CO | $CH_3$ | $CH_2Ph$ | |
| 2.168 | 5,6-Dihydro-3-methyl-4H-thiopyran-2-yl | CO | $CH_3$ | $CH_3$ | |
| 2.169 | 5,6-Dihydro-3-methyl-4H-thiopyran-2-yl | CO | $CH_3$ | $CH_2CH_3$ | |
| 2.170 | 5,6-Dihydro-3-methyl-4H-thiopyran-2-yl | CO | $CH_3$ | $CH(CH_3)_2$ | |
| 2.171 | 5,6-Dihydro-3-methyl-4H-thiopyran-2-yl | CO | $CH_3$ | $CH_2CH{=}CH_2$ | |
| 2.172 | 5,6-Dihydro-3-methyl-4H-thiopyran-2-yl | CO | $CH_3$ | $CH_2C{\equiv}CH$ | |
| 2.173 | 5,6-Dihydro-3-methyl-4H-thiopyran-2-yl | CO | $CH_3$ | $CH_2Ph$ | |
| 2.174 | Tetrahydrothiopyran-3-yl | CO | $CH_3$ | $CH_3$ | |
| 2.175 | Tetrahydrothiopyran-3-yl | CO | $CH_3$ | $CH_2CH_3$ | |

Tabelle 2 - Forts.

| Nr. | B | | A | $R^1$ | Physik. Daten Fp (°C) $^1$H-NMR (ppm) |
|---|---|---|---|---|---|
| 2.176 | Tetrahydrothiopyran-3-yl | CO | $CH_3$ | $CH(CH_3)_2$ | |
| 2.177 | Tetrahydrothiopyran-3-yl | CO | $CH_3$ | $CH_2CH=CH_2$ | |
| 2.178 | Tetrahydrothiopyran-3-yl | CO | $CH_3$ | $CH_2C\equiv CH$ | |
| 2.179 | Tetrahydrothiopyran-3-yl | CO | $CH_3$ | $CH_2Ph$ | |
| 2.180 | Tetrahydrothiopyran-4-yl | CO | $CH_3$ | $CH_3$ | |
| 2.181 | Tetrahydrothiopyran-4-yl | CO | $CH_3$ | $CH_2CH_3$ | |
| 2.182 | Tetrahydrothiopyran-4-yl | CO | $CH_3$ | $CH(CH_3)_2$ | |
| 2.183 | Tetrahydrothiopyran-4-yl | CO | $CH_3$ | $CH_2CH=CH_2$ | |
| 2.184 | Tetrahydrothiopyran-4-yl | CO | $CH_3$ | $CH_2C\equiv CH$ | |
| 2.185 | Tetrahydrothiopyran-4-yl | CO | $CH_3$ | $CH_2Ph$ | |
| 2.186 | Phenyl | CO | $CH_3$ | $CH_3$ | |
| 2.187 | Phenyl | CO | $CH_3$ | $CH_2CH_3$ | |
| 2.188 | Phenyl | CO | $CH_3$ | $CH(CH_3)_2$ | |
| 2.189 | Phenyl | CO | $CH_3$ | $CH_2CH=CH_2$ | |
| 2.190 | Phenyl | CO | $CH_3$ | $CH_2C\equiv CH$ | |
| 2.191 | Phenyl | CO | $CH_3$ | $CH_2Ph$ | |
| 2.192 | Phenyl | CO | $CH(CH_3)_2$ | $CH_3$ | |
| 2.193 | Phenyl | CO | $C(CH_3)_3$ | $CH_3$ | |
| 2.194 | Phenyl | $SO_2$ | $CH_3$ | $CH_3$ | |
| 2.195 | Phenyl | $SO_2$ | $CH_3$ | $CH_2CH_3$ | |
| 2.196 | Phenyl | $SO_2$ | $CH_3$ | $CH(CH_3)_2$ | |
| 2.197 | Phenyl | $SO_2$ | $CH_3$ | $CH_2CH=CH_2$ | |
| 2.198 | Phenyl | $SO_2$ | $CH_3$ | $CH_2C\equiv CH$ | |
| 2.199 | Phenyl | $SO_2$ | $CH_3$ | $CH_2Ph$ | |
| 2.200 | 2-Methylphenyl | CO | $CH_3$ | $CH_3$ | |
| 2.201 | 2-Methylphenyl | CO | $CH_3$ | $CH_2CH_3$ | |
| 2.202 | 2-Methylphenyl | CO | $CH_3$ | $CH(CH_3)_2$ | |

Tabelle 2 - Forts.

| Nr. | B | | A | $R^1$ | Physik. Daten Fp (°C) $^1$H-NMR (ppm) |
|---|---|---|---|---|---|
| 2.203 | 2-Methylphenyl | CO | $CH_3$ | $CH_2CH=CH_2$ | |
| 2.204 | 2-Methylphenyl | CO | $CH_3$ | $CH_2C\equiv CH$ | |
| 2.205 | 2-Methylphenyl | CO | $CH_3$ | $CH_2Ph$ | |
| 2.206 | 2-Methylphenyl | $SO_2$ | $CH_3$ | $CH_3$ | |
| 2.207 | 2-Methylphenyl | $SO_2$ | $CH_3$ | $CH_2CH_3$ | |
| 2.208 | 2-Methylphenyl | $SO_2$ | $CH_3$ | $CH(CH_3)_2$ | |
| 2.209 | 2-Methylphenyl | $SO_2$ | $CH_3$ | $CH_2CH=CH_2$ | |
| 2.210 | 2-Methylphenyl | $SO_2$ | $CH_3$ | $CH_2C\equiv CH$ | |
| 2.211 | 2-Methylphenyl | $SO_2$ | $CH_3$ | $CH_2Ph$ | |
| 2.212 | 3-Methylphenyl | CO | $CH_3$ | $CH_3$ | |
| 2.213 | 4-Methylphenyl | CO | $CH_3$ | $CH_3$ | |
| 2.214 | 2,4-Dimethylphenyl | CO | $CH_3$ | $CH_3$ | |
| 2.215 | 2,4-Dimethylphenyl | CO | $CH_3$ | $CH_2CH_3$ | |
| 2.216 | 2,4-Dimethylphenyl | CO | $CH_3$ | $CH(CH_3)_2$ | |
| 2.217 | 2,4-Dimethylphenyl | CO | $CH_3$ | $CH_2CH=CH_2$ | |
| 2.218 | 2,4-Dimethylphenyl | CO | $CH_3$ | $CH_2C\equiv CH$ | |
| 2.219 | 2,4-Dimethylphenyl | CO | $CH_3$ | $CH_2Ph$ | |
| 2.220 | 2,4-Dimethylphenyl | $SO_2$ | $CH_3$ | $CH_3$ | |
| 2.221 | 2,4-Dimethylphenyl | $SO_2$ | $CH_3$ | $CH_2CH_3$ | |
| 2.222 | 2,4-Dimethylphenyl | $SO_2$ | $CH_3$ | $CH(CH_3)_2$ | |
| 2.223 | 2,4-Dimethylphenyl | $SO_2$ | $CH_3$ | $CH_2CH=CH_2$ | |
| 2.224 | 2,4-Dimethylphenyl | $SO_2$ | $CH_3$ | $CH_2C\equiv CH$ | |
| 2.225 | 2,4-Dimethylphenyl | $SO_2$ | $CH_3$ | $CH_2Ph$ | |
| 2.226 | 2,6-Dimethylphenyl | CO | $CH_3$ | $CH_3$ | |
| 2.227 | 2,6-Dimethylphenyl | CO | $CH_3$ | $CH_2CH_3$ | |
| 2.228 | 2,6-Dimethylphenyl | CO | $CH_3$ | $CH(CH_3)_2$ | |
| 2.229 | 2,6-Dimethylphenyl | CO | $CH_3$ | $CH_2CH=CH_2$ | |
| 2.230 | 2,6-Dimethylphenyl | CO | $CH_3$ | $CH_2C\equiv CH$ | |
| 2.231 | 2,6-Dimethylphenyl | CO | $CH_3$ | $CH_2Ph$ | |
| 2.232 | 2,6-Dimethylphenyl | $SO_2$ | $CH_3$ | $CH_3$ | |
| 2.233 | 2,6-Dimethylphenyl | $SO_2$ | $CH_3$ | $CH_2CH_3$ | |
| 2.234 | 2,6-Dimethylphenyl | $SO_2$ | $CH_3$ | $CH(CH_3)_2$ | |
| 2.235 | 2,6-Dimethylphenyl | $SO_2$ | $CH_3$ | $CH_2CH=CH_2$ | |
| 2.236 | 2,6-Dimethylphenyl | $SO_2$ | $CH_3$ | $CH_2C\equiv CH$ | |
| 2.237 | 2,6-Dimethylphenyl | $SO_2$ | $CH_3$ | $CH_2Ph$ | |
| 2.238 | 2-Trifluormethylphenyl | CO | $CH_3$ | $CH_3$ | |
| 2.239 | 2-Trifluormethylphenyl | CO | $CH_3$ | $CH_2CH_3$ | |

Tabelle 2 - Forts.

| Nr. | B | | A | $R^1$ | Physik. Daten Fp (°C) $^1H$-NMR (ppm) |
|---|---|---|---|---|---|
| 2.240 | 2-Trifluormethylphenyl | CO | $CH_3$ | $CH(CH_3)_2$ | |
| 2.241 | 2-Trifluormethylphenyl | CO | $CH_3$ | $CH_2CH=CH_2$ | |
| 2.242 | 2-Trifluormethylphenyl | CO | $CH_3$ | $CH_2C\equiv CH$ | |
| 2.243 | 2-Trifluormethylphenyl | CO | $CH_3$ | $CH_2Ph$ | |
| 2.244 | 2-Trifluormethylphenyl | $SO_2$ | $CH_3$ | $CH_3$ | |
| 2.245 | 2-Methoxyphenyl | CO | $CH_3$ | $CH_3$ | |
| 2.246 | 2-Methoxyphenyl | CO | $CH_3$ | $CH_2CH_3$ | |
| 2.247 | 2-Methoxyphenyl | CO | $CH_3$ | $CH(CH_3)_2$ | |
| 2.248 | 2-Methoxyphenyl | CO | $CH_3$ | $CH_2CH=CH_2$ | |
| 2.249 | 2-Methoxyphenyl | CO | $CH_3$ | $CH_2C\equiv CH$ | |
| 2.250 | 2-Methoxyphenyl | CO | $CH_3$ | $CH_2Ph$ | |
| 2.251 | 2-Methoxyphenyl | $SO_2$ | $CH_3$ | $CH_3$ | |
| 2.252 | 2,6-Dimethoxyphenyl | CO | $CH_3$ | $CH_3$ | |
| 2.253 | 2,6-Dimethoxyphenyl | CO | $CH_3$ | $CH_2CH_3$ | |
| 2.254 | 2,6-Dimethoxyphenyl | CO | $CH_3$ | $CH(CH_3)_2$ | |
| 2.255 | 2,6-Dimethoxyphenyl | CO | $CH_3$ | $CH_2CH=CH_2$ | |
| 2.256 | 2,6-Dimethoxyphenyl | CO | $CH_3$ | $CH_2C\equiv CH$ | |
| 2.257 | 2,6-Dimethoxyphenyl | CO | $CH_3$ | $CH_2Ph$ | |
| 2.258 | 2,6-Dimethoxyphenyl | $SO_2$ | $CH_3$ | $CH_3$ | |
| 2.259 | 2-Trifluormethoxyphenyl | CO | $CH_3$ | $CH_3$ | |
| 2.260 | 2-Trifluormethoxyphenyl | CO | $CH_3$ | $CH_2CH_3$ | |
| 2.261 | 2-Trifluormethoxyphenyl | CO | $CH_3$ | $CH(CH_3)_2$ | |
| 2.262 | 2-Trifluormethoxyphenyl | CO | $CH_3$ | $CH_2CH=CH_2$ | |
| 2.263 | 2-Trifluormethoxyphenyl | CO | $CH_3$ | $CH_2C\equiv CH$ | |
| 2.264 | 2-Trifluormethoxyphenyl | CO | $CH_3$ | $CH_2Ph$ | |
| 2.265 | 2-Methylthiophenyl | CO | $CH_3$ | $CH_3$ | |
| 2.266 | 2-Methylsulfinylphenyl | CO | $CH_3$ | $CH_3$ | |
| 2.267 | 2-Methylsulfonylphenyl | CO | $CH_3$ | $CH_3$ | |
| 2.268 | 2-Fluorphenyl | CO | $CH_3$ | $CH_3$ | |
| 2.269 | 2-Fluorphenyl | CO | $CH_3$ | $CH_2CH_3$ | |
| 2.270 | 2-Fluorphenyl | CO | $CH_3$ | $CH(CH_3)_2$ | |
| 2.271 | 2-Fluorphenyl | CO | $CH_3$ | $CH_2CH=CH_2$ | 75- 77 |
| 2.272 | 2-Fluorphenyl | CO | $CH_3$ | $CH_2C\equiv CH$ | |
| 2.273 | 2-Fluorphenyl | CO | $CH_3$ | $CH_2Ph$ | 98-100 |
| 2.274 | 2-Fluorphenyl | $SO_2$ | $CH_3$ | $CH_3$ | |
| 2.275 | 2-Fluorphenyl | $SO_2$ | $CH_3$ | $CH_2CH_3$ | |
| 2.276 | 2-Fluorphenyl | $SO_2$ | $CH_3$ | $CH(CH_3)_2$ | |
| 2.277 | 2-Fluorphenyl | $SO_2$ | $CH_3$ | $CH_2CH=CH_2$ | |

Tabelle 2 - Forts.

| Nr. | B | | A | $R^1$ | Physik. Daten Fp (°C) $^1H$-NMR (ppm) |
|---|---|---|---|---|---|
| 2.278 | 2-Fluorphenyl | $SO_2$ | $CH_3$ | $CH_2C{\equiv}CH$ | |
| 2.279 | 2-Fluorphenyl | $SO_2$ | $CH_3$ | $CH_2Ph$ | |
| 2.280 | 2-Fluorphenyl | CO | $CH_2CF_3$ | $CH_3$ | |
| 2.281 | 2-Fluorphenyl | CO | $CH_2CF_3$ | $CH_2CH_3$ | |
| 2.282 | 2-Fluorphenyl | CO | $CH_2CF_3$ | $CH(CH_3)_2$ | |
| 2.283 | 2-Fluorphenyl | CO | $CH_2CF_3$ | $CH_2CH{=}CH_2$ | |
| 2.284 | 2-Fluorphenyl | CO | $CH_2CF_3$ | $CH_2C{\equiv}CH$ | |
| 2.285 | 2-Fluorphenyl | CO | $CH_2CF_3$ | $CH_2Ph$ | |
| 2.286 | 2,3-Difluorphenyl | CO | $CH_3$ | $CH_3$ | |
| 2.287 | 2,3-Difluorphenyl | CO | $CH_3$ | $CH_2CH_3$ | |
| 2.288 | 2,3-Difluorphenyl | CO | $CH_3$ | $CH(CH_3)_2$ | |
| 2.289 | 2,3-Difluorphenyl | CO | $CH_3$ | $CH_2CH{=}CH_2$ | |
| 2.290 | 2,3-Difluorphenyl | CO | $CH_3$ | $CH_2C{\equiv}CH$ | |
| 2.291 | 2,3-Difluorphenyl | CO | $CH_3$ | $CH_2Ph$ | |
| 2.292 | 2,4-Difluorphenyl | CO | $CH_3$ | $CH_3$ | |
| 2.293 | 2,4-Difluorphenyl | CO | $CH_3$ | $CH_2CH_3$ | |
| 2.294 | 2,4-Difluorphenyl | CO | $CH_3$ | $CH(CH_3)_2$ | |
| 2.295 | 2,4-Difluorphenyl | CO | $CH_3$ | $CH_2CH{=}CH_2$ | |
| 2.296 | 2,4-Difluorphenyl | CO | $CH_3$ | $CH_2C{\equiv}CH$ | |
| 2.297 | 2,4-Difluorphenyl | CO | $CH_3$ | $CH_2Ph$ | |
| 2.298 | 2,5-Difluorphenyl | CO | $CH_3$ | $CH_3$ | |
| 2.299 | 2,5-Difluorphenyl | CO | $CH_3$ | $CH_2CH_3$ | |
| 2.300 | 2,5-Difluorphenyl | CO | $CH_3$ | $CH(CH_3)_2$ | |
| 2.301 | 2,5-Difluorphenyl | CO | $CH_3$ | $CH_2CH{=}CH_2$ | |
| 2.302 | 2,5-Difluorphenyl | CO | $CH_3$ | $CH_2C{\equiv}CH$ | |
| 2.303 | 2,5-Difluorphenyl | CO | $CH_3$ | $CH_2Ph$ | |
| 2.304 | 2,6-Difluorphenyl | CO | $CH_3$ | $CH_3$ | |
| 2.305 | 2,6-Difluorphenyl | CO | $CH_3$ | $CH_2CH_3$ | |
| 2.306 | 2,6-Difluorphenyl | CO | $CH_3$ | $CH(CH_3)_2$ | |
| 2.307 | 2,6-Difluorphenyl | CO | $CH_3$ | $CH_2CH{=}CH_2$ | |
| 2.308 | 2,6-Difluorphenyl | CO | $CH_3$ | $CH_2C{\equiv}CH$ | |
| 2.309 | 2,6-Difluorphenyl | CO | $CH_3$ | $CH_2Ph$ | |
| 2.310 | 2-Chlorphenyl | CO | $CH_3$ | $CH_3$ | |
| 2.311 | 2-Chlorphenyl | CO | $CH_3$ | $CH_2CH_3$ | |
| 2.312 | 2-Chlorphenyl | CO | $CH_3$ | $CH(CH_3)_2$ | |
| 2.313 | 2-Chlorphenyl | CO | $CH_3$ | $CH_2CH{=}CH_2$ | |
| 2.314 | 2-Chlorphenyl | CO | $CH_3$ | $CH_2C{\equiv}CH$ | |
| 2.315 | 2-Chlorphenyl | CO | $CH_3$ | $CH_2Ph$ | |

Tabelle 2 - Forts.

| Nr. | B | A | | $R^1$ | Physik. Daten Fp (°C) $^1$H-NMR (ppm) |
|---|---|---|---|---|---|
| 2.316 | 2-Chlorphenyl | $SO_2$ | $CH_3$ | $CH_3$ | |
| 2.317 | 2-Chlorphenyl | $SO_2$ | $CH_3$ | $CH_2CH_3$ | |
| 2.318 | 2-Chlorphenyl | $SO_2$ | $CH_3$ | $CH(CH_3)_2$ | |
| 2.319 | 2-Chlorphenyl | $SO_2$ | $CH_3$ | $CH_2CH=CH_2$ | |
| 2.320 | 2-Chlorphenyl | $SO_2$ | $CH_3$ | $CH_2C{\equiv}CH$ | |
| 2.321 | 2-Chlorphenyl | $SO_2$ | $CH_3$ | $CH_2Ph$ | |
| 2.322 | 2-Chlorphenyl | CO | $CH(CH_3)_2$ | $CH_3$ | |
| 2.323 | 2-Chlorphenyl | CO | $C(CH_3)_3$ | $CH_3$ | |
| 2.324 | 2-Chlorphenyl | CO | $CH_2CF_3$ | $CH_3$ | |
| 2.325 | 2-Chlorphenyl | CO | $CH_2CF_3$ | $CH_2CH_3$ | |
| 2.326 | 2-Chlorphenyl | CO | $CH_2CF_3$ | $CH(CH_3)_2$ | |
| 2.327 | 2-Chlorphenyl | CO | $CH_2CF_3$ | $CH_2CH=CH_2$ | |
| 2.328 | 2-Chlorphenyl | CO | $CH_2CF_3$ | $CH_2C{\equiv}CH$ | |
| 2.329 | 2-Chlorphenyl | CO | $CH_2CF_3$ | $CH_2Ph$ | |
| 2.330 | 3-Chlorphenyl | CO | $CH_3$ | $CH_3$ | |
| 2.331 | 3-Chlorphenyl | $SO_2$ | $CH_3$ | $CH_3$ | |
| 2.332 | 4-Chlorphenyl | CO | $CH_3$ | $CH_3$ | |
| 2.333 | 4-Chlorphenyl | $SO_2$ | $CH_3$ | $CH_3$ | |
| 2.334 | 2,3-Dichlorphenyl | CO | $CH_3$ | $CH_3$ | |
| 2.335 | 2,3-Dichlorphenyl | CO | $CH_3$ | $CH_2CH_3$ | |
| 2.336 | 2,3-Dichlorphenyl | CO | $CH_3$ | $CH(CH_3)_2$ | |
| 2.337 | 2,3-Dichlorphenyl | CO | $CH_3$ | $CH_2CH=CH_2$ | |
| 2.338 | 2,3-Dichlorphenyl | CO | $CH_3$ | $CH_2C{\equiv}CH$ | |
| 2.339 | 2,3-Dichlorphenyl | CO | $CH_3$ | $CH_2Ph$ | |
| 2.340 | 2,4-Dichlorphenyl | CO | $CH_3$ | $CH_3$ | |
| 2.341 | 2,4-Dichlorphenyl | CO | $CH_3$ | $CH_2CH_3$ | |
| 2.342 | 2,4-Dichlorphenyl | CO | $CH_3$ | $CH(CH_3)_2$ | |
| 2.343 | 2,4-Dichlorphenyl | CO | $CH_3$ | $CH_2CH=CH_2$ | |
| 2.344 | 2,4-Dichlorphenyl | CO | $CH_3$ | $CH_2C{\equiv}CH$ | |
| 2.345 | 2,4-Dichlorphenyl | CO | $CH_3$ | $CH_2Ph$ | |
| 2.346 | 2,5-Dichlorphenyl | CO | $CH_3$ | $CH_3$ | |
| 2.347 | 2,5-Dichlorphenyl | CO | $CH_3$ | $CH_2CH_3$ | |
| 2.348 | 2,5-Dichlorphenyl | CO | $CH_3$ | $CH(CH_3)_2$ | |
| 2.349 | 2,5-Dichlorphenyl | CO | $CH_3$ | $CH_2CH=CH_2$ | |
| 2.350 | 2,5-Dichlorphenyl | CO | $CH_3$ | $CH_2C{\equiv}CH$ | |
| 2.351 | 2,5-Dichlorphenyl | CO | $CH_3$ | $CH_2Ph$ | |
| 2.352 | 2,6-Dichlorphenyl | CO | $CH_3$ | $CH_3$ | |
| 2.353 | 2,6-Dichlorphenyl | CO | $CH_3$ | $CH_2CH_3$ | |

Tabelle 2 - Forts.

| Nr. | B | | A | $R^1$ | Physik. Daten Fp (°C) $^1$H-NMR (ppm) |
|---|---|---|---|---|---|
| 2.354 | 2,6-Dichlorphenyl | CO | $CH_3$ | $CH(CH_3)_2$ | |
| 2.355 | 2,6-Dichlorphenyl | CO | $CH_3$ | $CH_2CH=CH_2$ | |
| 2.356 | 2,6-Dichlorphenyl | CO | $CH_3$ | $CH_2C≡CH$ | |
| 2.357 | 2,6-Dichlorphenyl | CO | $CH_3$ | $CH_2Ph$ | |
| 2.358 | 2,6-Dichlorphenyl | $SO_2$ | $CH_3$ | $CH_3$ | |
| 2.359 | 2,6-Dichlorphenyl | $SO_2$ | $CH_3$ | $CH_2CH_3$ | |
| 2.360 | 2,6-Dichlorphenyl | $SO_2$ | $CH_3$ | $CH(CH_3)_2$ | |
| 2.361 | 2,6-Dichlorphenyl | $SO_2$ | $CH_3$ | $CH_2CH=CH_2$ | |
| 2.362 | 2,6-Dichlorphenyl | $SO_2$ | $CH_3$ | $CH_2C≡CH$ | |
| 2.363 | 2,6-Dichlorphenyl | $SO_2$ | $CH_3$ | $CH_2Ph$ | |
| 2.364 | 2,4,6-Trichlorphenyl | CO | $CH_3$ | $CH_3$ | |
| 2.365 | 2,4,6-Trichlorphenyl | CO | $CH_3$ | $CH_2CH_3$ | |
| 2.366 | 2,4,6-Trichlorphenyl | CO | $CH_3$ | $CH(CH_3)_2$ | |
| 2.367 | 2,4,6-Trichlorphenyl | CO | $CH_3$ | $CH_2CH=CH_2$ | |
| 2.368 | 2,4,6-Trichlorphenyl | CO | $CH_3$ | $CH_2C≡CH$ | |
| 2.369 | 2,4,6-Trichlorphenyl | CO | $CH_3$ | $CH_2Ph$ | |
| 2.370 | 2-Chlor-6-methylphenyl | CO | $CH_3$ | $CH_3$ | |
| 2.371 | 2-Chlor-6-methylphenyl | CO | $CH_3$ | $CH_2CH_3$ | |
| 2.372 | 2-Chlor-6-methylphenyl | CO | $CH_3$ | $CH(CH_3)_2$ | |
| 2.373 | 2-Chlor-6-methylphenyl | CO | $CH_3$ | $CH_2CH=CH_2$ | |
| 2.374 | 2-Chlor-6-methylphenyl | CO | $CH_3$ | $CH_2C≡CH$ | |
| 2.375 | 2-Chlor-6-methlyphenyl | CO | $CH_3$ | $CH_2Ph$ | |
| 2.376 | 2-Chlor-6-methylphenyl | $SO_2$ | $CH_3$ | $CH_3$ | |
| 2.377 | 2-Chlor-6-methylphenyl | $SO_2$ | $CH_3$ | $CH_2CH_3$ | |
| 2.378 | 2-Chlor-6-methylphenyl | $SO_2$ | $CH_3$ | $CH(CH_3)_2$ | |
| 2.379 | 2-Chlor-6-methylphenyl | $SO_2$ | $CH_3$ | $CH_2CH=CH_2$ | |
| 2.380 | 2-Chlor-6-methylphenyl | $SO_2$ | $CH_3$ | $CH_2C≡CH$ | |
| 2.381 | 2-Chlor-6-methylphenyl | $SO_2$ | $CH_3$ | $CH_2Ph$ | |
| 2.382 | 2-Bromphenyl | CO | $CH_3$ | $CH_3$ | 3,23(s)3H;3,63(s)3H; 7,32(m)4H;7,58(m)1H; 7,82(d)1H;J=5Hz |
| 2.383 | 2-Bromphenyl | CO | $CH_3$ | $CH_2CH_3$ | |
| 2.384 | 2-Bromphenyl | CO | $CH_3$ | $CH(CH_3)_2$ | |
| 2.385 | 2-Bromphenyl | CO | $CH_3$ | $CH_2CH=CH_2$ | |
| 2.386 | 2-Bromphenyl | CO | $CH_3$ | $CH_2C≡CH$ | |
| 2.387 | 2-Bromphenyl | CO | $CH_3$ | $CH_2Ph$ | |
| 2.388 | 2-Cyanophenyl | CO | $CH_3$ | $CH_3$ | |
| 2.389 | 2-Cyanophenyl | CO | $CH_3$ | $CH_2CH_3$ | |

Tabelle 2 - Forts.

| Nr. | B | A | $R^1$ | Physik. Daten Fp (°C) $^1$H-NMR (ppm) |
|---|---|---|---|---|
| 2.390 | 2-Cyanophenyl | CO | $CH_3$ | $CH(CH_3)_2$ | |
| 2.391 | 2-Cyanophenyl | CO | $CH_3$ | $CH_2CH=CH_2$ | |
| 2.392 | 2-Cyanophenyl | CO | $CH_3$ | $CH_2C\equiv CH$ | |
| 2.393 | 2-Cyanophenyl | CO | $CH_3$ | $CH_2Ph$ | |
| 2.394 | 2-Cyanophenyl | $SO_2$ | $CH_3$ | $CH_3$ | |
| 2.395 | 2-Nitrophenyl | CO | $CH_3$ | $CH_3$ | |
| 2.396 | 2-Nitrophenyl | CO | $CH_3$ | $CH_2CH_3$ | |
| 2.397 | 2-Nitrophenyl | $SO_2$ | $CH_3$ | $CH_2CH_3$ | |
| 2.398 | 2-Nitrophenyl | CO | $CH_3$ | $CH(CH_3)_2$ | |
| 2.399 | 2-Nitrophenyl | CO | $CH_3$ | $CH_2CH=CH_2$ | |
| 2.400 | 2-Nitrophenyl | CO | $CH_3$ | $CH_2C\equiv CH$ | |
| 2.401 | 2-Nitrophenyl | CO | $CH_3$ | $CH_2Ph$ | |
| 2.402 | 2-Nitrophenyl | $SO_2$ | $CH_3$ | $CH_3$ | |
| 2.403 | 2-Carbomethoxyphenyl | CO | $CH_3$ | $CH_3$ | |
| 2.404 | 2-Carbomethoxyphenyl | CO | $CH_3$ | $CH_2CH_3$ | |
| 2.405 | 2-Carbomethoxyphenyl | CO | $CH_3$ | $CH(CH_3)_2$ | |
| 2.406 | 2-Carbomethoxyphenyl | CO | $CH_3$ | $CH_2CH=CH_2$ | |
| 2.407 | 2-Carbomethoxyphenyl | CO | $CH_3$ | $CH_2C\equiv CH$ | |
| 2.408 | 2-Carbomethoxyphenyl | CO | $CH_3$ | $CH_2Ph$ | |
| 2.409 | 2-Carbomethoxyphenyl | $SO_2$ | $CH_3$ | $CH_3$ | |
| 2.410 | 2-Carbethoxyphenyl | CO | $CH_3$ | $CH_3$ | |
| 2.411 | 2-Carbethoxyphenyl | $SO_2$ | $CH_3$ | $CH_3$ | |
| 2.412 | 2-(N,N-Dimethylcarbamido)phenyl | CO | $CH_3$ | $CH_3$ | |
| 2.413 | 2-Pyridyl | CO | $CH_3$ | $CH_3$ | |
| 2.414 | 3-Pyridyl | CO | $CH_3$ | $CH_3$ | |
| 2.415 | 2-Fluor-3-pyridyl | CO | $CH_3$ | $CH_3$ | |
| 2.416 | 2-Chlor-3-pyridyl | CO | $CH_3$ | $CH_3$ | |
| 2.417 | 4-Chlor-3-pyridyl | CO | $CH_3$ | $CH_3$ | |
| 2.418 | 4-Pyridyl | CO | $CH_3$ | $CH_3$ | |
| 2.419 | 5-Pyrimidyl | CO | $CH_3$ | $CH_3$ | |
| 2.420 | 1-Naphthyl | CO | $CH_3$ | $CH_3$ | |
| 2.421 | 2-Naphthyl | CO | $CH_3$ | $CH_3$ | |
| 2.422 | 2-Chinolinyl | CO | $CH_3$ | $CH_3$ | |
| 2.423 | 3-Chinolinyl | CO | $CH_3$ | $CH_3$ | |
| 2.424 | 2-Methyl-4-chinolinyl- | CO | $CH_3$ | $CH_3$ | |
| 2.425 | 3,7-Dichlor-8-chinolinyl | CO | $CH_3$ | $CH_3$ | |

Tabelle 2 - Forts.

| Nr. | B | | A | R1 | Physik. Daten Fp (°C) $^1$H-NMR (ppm) |
|---|---|---|---|---|---|
| 2.426 | 1-Methyl-2-pyrolyl | CO | $CH_3$ | $CH_3$ | |
| 2.427 | 2-Furanyl | CO | $CH_3$ | $CH_3$ | |
| 2.428 | 3-Methyl-2-furanyl | CO | $CH_3$ | $CH_3$ | |
| 2.429 | 5-Brom-2-furanyl | CO | $CH_3$ | $CH_3$ | |
| 2.430 | 5-Methyl-2-furanyl | CO | $CH_3$ | $CH_3$ | |
| 2.431 | 3-Furanyl | CO | $CH_3$ | $CH_3$ | |
| 2.432 | 2,5-Dimethyl-3-furanyl | CO | $CH_3$ | $CH_3$ | |
| 2.433 | 2,4,5-Trimethyl-3-furanyl | CO | $CH_3$ | $CH_3$ | |
| 2.434 | 2-Thienyl | CO | $CH_3$ | $CH_3$ | |
| 2.435 | 2-Thienyl | CO | $CH_3$ | $CH_2CH_3$ | |
| 2.436 | 2-Thienyl | CO | $CH_3$ | $CH(CH_3)_2$ | |
| 2.437 | 2-Thienyl | CO | $CH_3$ | $CH_2CH=CH_2$ | |
| 2.438 | 2-Thienyl | CO | $CH_3$ | $CH_2C\equiv CH$ | |
| 2.439 | 2-Thienyl | CO | $CH_3$ | $CH_2Ph$ | |
| 2.440 | 3-Chlor-2-thienyl | CO | $CH_3$ | $CH_3$ | 3,24(s)3H;3.60(s) 3H;7,32;7,56;7,64; 7,87; je (d) 1H; J=4Hz |
| 2.441 | 5-Methyl-2-thienyl | CO | $CH_3$ | $CH_3$ | |
| 2.442 | 3-Thienyl | CO | $CH_3$ | $CH_3$ | |
| 2.443 | 4-Chlor-3-thienyl | CO | $CH_3$ | $CH_3$ | 168-171 |
| 2.444 | 1-Methyl-5-pyrazolyl | CO | $CH_3$ | $CH_3$ | |
| 2.445 | 1-Methyl-4-pyrazolyl | CO | $CH_3$ | $CH_3$ | |
| 2.446 | 1-Methyl-2-imidazolyl | CO | $CH_3$ | $CH_3$ | |
| 2.447 | 5-Isoxazolyl | CO | $CH_3$ | $CH_3$ | |
| 2.448 | 4-Isoxazolyl | CO | $CH_3$ | $CH_3$ | |
| 2.449 | 3-Isopropyl-5-isoxazolyl | CO | $CH_3$ | $CH_3$ | |
| 2.450 | 3-Methyl-4-isoxazolyl | CO | $CH_3$ | $CH_3$ | |
| 2.451 | 4-Isothiazolyl | CO | $CH_3$ | $CH_3$ | |

Anwendungsbeispiele

Die Wirkung der 5-Amino-6-pyridazonderivate I auf das Wachstum der Testpflanzen ließ sich durch folgende Gewächshausversuche zeigen.

Als Kulturgefäße dienten Plastikblumentöpfe mit 300 $cm^3$ Inhalt und lehmigem Sand mit etwa 3 % Humus als Substrat. Die Samen der Testpflanzen wurden nach Arten getrennt eingesät.

Zum Zwecke der Nachauflaufbehandlung wurden entweder direkt gesäte oder in den gleichen Gefäßen aufgewachsene Pflanzen ausgewählt oder sie wurden erst als Keimpflanzen getrennt angezogen und einige Tage vor der Behandlung in die Versuchsgefäße verpflanzt.

Je nach Wuchsform wurden die Testpflanzen bei einer Wuchshöhe von 3 bis 15 cm dann mit den in Wasser als Verteilungsmittel suspendierten oder emulgierten Wirkstoffen, die durch fein verteilende Düsen

gespritzt wurden, behandelt. Die Aufwandmengen für die Nachauflaufbehandlung betrug 0,25 kg Wirkstoff/ha.

Die Versuchsgefäße wurden im Gewächshaus aufgestellt, wobei für wärmeliebende Arten wärmere Bereiche (20 bis 35 °C) und für solche gemäßigter Klimate 10 bis 20 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Chenopodium album | Weißer Gänsefuß |
| Stellaria media | Vogelsternmiere |
| Triticum aestivum | Winterweizen |

Mit 0,250 kg Wirkstoff/ha im Nachauflaufverfahren eingesetzt, lassen sich mit Beispiel 1.036 bzw. Beispiel 1.039 und mit Beispiel 1.065 unerwünschte Pflanzen sehr gut bekämpfen, und zwar mit gleichzeitiger Verträglichkeit für Weizen.

Mit 0,125 kg Wirkstoff/ha im Nachauflaufverfahren eingesetzt, lassen sich mit Beispiel 1.123 unerwünschte Pflanzen sehr gut bekämpfen, und zwar mit gleichzeitiger guter Verträglichkeit für Weizen.

## Patentansprüche

**1.** 5-Amino-6-pyridazonderivate der allgemeinen Formeln Ia und Ib

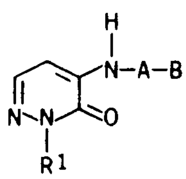 Ia

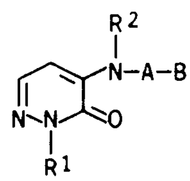 Ib

in denen die Substituenten folgende Bedeutung haben:

$R^1$
- eine $C_1$-$C_4$-Alkylgruppe, welche bis zu drei Halogenatome tragen kann,

$R^2$
- eine $C_1$-$C_4$-Alkylgruppe oder eine $C_3$-$C_6$-Alkenylgruppe, wobei diese Gruppen bis zu zwei Phenylreste tragen können oder
- eine $C_3$-$C_6$-Alkinylgruppe,

A -CO- oder $-SO_2-$

B
- eine $C_1$-$C_6$-Alkylgruppe, eine $C_2$-$C_6$-Alkenylgruppe oder eine $C_2$-$C_6$-Alkinylgruppe, wobei diese Gruppen bis zu drei der folgenden Reste tragen können: Halogenatome, $C_1$-$C_4$-Alkoxygruppen, $C_1$-$C_4$-Alkylthiogruppen und/oder Phenylreste,
- eine iso- oder heterocyclische $C_3$-$C_7$-Cycloalkylgruppe oder eine iso- oder heterocyclische $C_3$-$C_7$-Cycloalkenylgruppe, wobei diese Gruppen mit ein bis zwei Cycloalkylgruppen dieser Art, oder mit ein bis zwei Benzolkernen anelliert sein können, wobei die Gesamtzahl der Ringglieder 3 bis 16 beträgt und diese cyclischen Reste bis zu drei der folgenden Gruppen tragen können: Halogenatome, $C_1$-$C_4$-Alkylgruppen, $C_2$-$C_6$-Alkenylgruppen, $C_2$-$C_6$-Alkinylgruppen und/oder Phenylreste,
- ein 1 bis 3 kerniger aromatischer oder heteroaromatischer Rest, welcher bis zu drei der folgenden Gruppen tragen kann: $C_1$-$C_4$-Alkylgruppen, $C_1$-$C_4$-Halogenalkylgruppen, $C_1$-$C_4$-Alkoxygruppen, $C_1$-$C_4$-Halogenalkoxygruppen, $C_1$-$C_4$-Alkylthiogruppen, $C_1$-$C_4$-Alkylsulfinylgruppen, $C_1$-$C_4$-Alkylsulfonylgruppen, Cyanogruppen, Nitrogruppen, Carbo-$C_1$-$C_4$-alkoxygruppen, N,N-Di-$C_1$-$C_4$-alkylcarbamidogruppen und/oder Halogenatome.

2. Verfahren zur Herstellung der Verbindungen Ia gemäß Anspruch 1, dadurch gekennzeichnet, daß man ein Pyridazon der allgemeinen Formel II

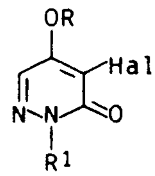

II

in der R eine Alkylgruppe und Hal Halogen bedeuten, mit Hydrazin in das 5-Amino-6-pyridazon III

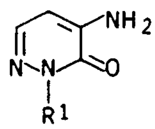

III

überführt und dieses anschließend mit einer Verbindung der allgemeinen Formel IV

B-A-X IV

in der X Halogen oder Carboxylat bedeutet, zu Ia umsetzt.

3. Verfahren zur Herstellung der Verbindungen Ib gemäß Anspruch 1, dadurch gekennzeichnet, daß man ein 5-Amino-6-pyridazonderivat der Formel Ia in Gegenwart einer Base mit einer Verbindung der allgemeinen Formel V

$R^2$-Y V

in der Y Halogen, eine Sulfonat- oder eine Sulfatgruppe bedeutet, zu Ib umsetzt.

4. Verwendung der substituierten Pyridazonderivate Ia und/oder Ib gemäß Anspruch 1 als Herbizide.

5. Herbizide Mittel, gemäß Anspruch 1, enthaltend ein substituiertes Pyridazonderivat Ia und/oder Ib und inerte Zusatzstoffe.

6. Herbizide Mittel, gemäß Anspruch 5, enthaltend ein substituiertes Pyridazonderivat Ia und/oder Ib und weitere herbizide und/oder synergistisch wirkende Bestandteile.

7. Verfahren zur Bekämpfung unerwünschten Pflanzenwuchses, dadurch gekennzeichnet, daß man die unerwünschten Pflanzen mit einer herbizid wirksamen Menge eines substituierten Pyridazonderivates Ia und/oder Ib gemäß Anspruch 1 behandelt.

## Claims

1. A 5-amino-6-pyridazone derivative of the general formula Ia or Ib

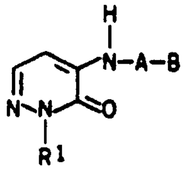

Ia

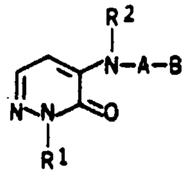

Ib

where $R^1$ is $C_1$-$C_4$-alkyl which may carry up to three halogen atoms,
$R^2$ is $C_1$-$C_4$-alkyl or $C_3$-$C_6$-alkenyl, where these groups may carry up to two phenyl radicals, or $C_3$-$C_6$-alkynyl,
A is -CO- or $-SO_2-$,
B is $C_1$-$C_6$-alkyl, $C_2$-$C_6$-alkenyl or $C_2$-$C_6$-alkynyl, where these groups may carry up to three of the following radicals: halogen, $C_1$-$C_4$-alkoxy, $C_1$-$C_4$-alkylthio and/or phenyl,
an isocyclic or heterocyclic $C_3$-$C_7$-cycloalkyl group or an isocyclic or heterocyclic $C_3$-$C_7$-cycloalkenyl group, where these groups may be fused with one or two cycloalkyl groups of this type or with one or two benzene nuclei, where the total number of ring members is from 3 to 16 and these cyclic radicals may carry up to three of the following groups: halogen, $C_1$-$C_4$-alkyl, $C_2$-$C_6$-alkenyl, $C_2$-$C_6$-alkynyl and/or phenyl,
a mononuclear to trinuclear aromatic or heteroaromatic radical which may carry up to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-alkylsulfinyl, $C_1 C_4$-alkylsulfonyl, cyano, nitro, carbo-$C_1$-$C_4$-alkoxy, N,N-di-$C_1$-$C_4$-alkylcarbamido and/or halogen.

2. A process for the preparation of a compound Ia as claimed in claim 1, wherein a pyridazone of the general formula II

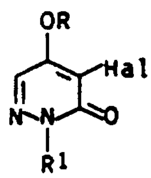

II

where R is alkyl and Hal is halogen, is converted with hydrazine into the 5-amino-6-pyridazone III

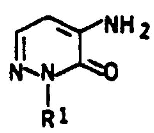

III

and the latter is then reacted with a compound of the general formula IV

B-A-X (IV)

where X is halogen or carboxylate, to give Ia.

3. A process for the preparation of a compound Ib as claimed in claim 1, wherein a 5-amino-6-pyridazone derivative of the formula Ia is reacted in the presence of a base with a compound of the general formula V

$R^2$-Y (V)

where Y is halogen, sulfonate or sulfate, to give Ib.

4. The use of the substituted pyridazone derivatives Ia and/or Ib as claimed in claim 1 as herbicides.

5. A herbicidal agent as claimed in claim 1, containing a substituted pyridazone derivative Ia and/or Ib and inert additives.

6. A herbicidal agent as claimed in claim 5, containing a substituted pyridazone derivative Ia and/or Ib and other herbicidal and/or synergistic components.

**7.** A method for controlling undesirable plant growth, wherein the undesirable plants are treated with a herbicidally effective amount of a substituted pyridazone derivative Ia and/or Ib as claimed in claim 1.

## Revendications

**1.** Dérivés de 5-amino-6-pyridazone des formules générales Ia et Ib

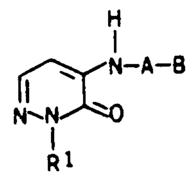

Ia

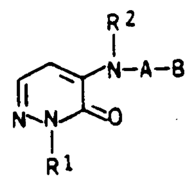

Ib

dans lesquelles les substituants ont la signification suivante :

$R^1$

- un groupe alkyle en C1-C4 qui peut porter jusqu'à trois atomes d'halogène

$R^2$

- un groupe alkyle en C1-C4 ou un groupe alcényle en C3-C6, ces groupes pouvant porter jusqu'à deux restes phényle ou un groupe alcynyle en C3-C6,

A

- -CO- ou $-SO_2-$

B

- un groupe alkyle en C1-C6, un groupe alcényle en C2-C6 ou un groupe alcynyle en C2-C6, ces groupes pouvant porter jusqu'à trois des restes suivants : atomes d'halogène, groupes alcoxy en C1-C4, groupes alkylthio en C1-C4 et/ou restes phényle
- un groupe cycloalkyle en C3-C7 iso ou hétérocyclique ou un groupe cycloalcényle en C3-C7 iso- ou hétérocyclique, ces groupes pouvant être condensés avec un à deux groupes cycloalkyle de ce type ou avec un à deux noyaux benzène, le nombre total des chaînons du noyau pouvant être de 3 à 16 et ces restes cycliques pouvant porter jusqu'à trois des groupes suivants : atomes d'halogène, groupes alkyle en C1-C4, groupes alcényle en C2-C6, groupes alcynyle en C2-C6 et/ou reste phényle,
- un reste aromatique ou hétéroaromatique de 1 à 3 noyaux, qui peut porter jusqu'à trois des groupes suivants : groupes alkyle en C1-C4, groupes halogénoalkyle en C1-C4, groupes alcoxy en C1-C4, groupes halogénoalcoxy en C1-C4, groupes alkylthio en C1-C4, groupes alkyle en C1-C4-sulfonyle, groupes alkyle en C1-C4-sulfonyle, groupes cyano, groupes nitro, groupes carbo-alcoxy en C1-C4, groupes N,N-di-alkyle en C1-C4-carbamido et/ou atomes d'halogène.

**2.** Procédé de préparation des composés Ia selon la revendication 1, caractérisé par le fait que l'on transforme une pyridazone de la formule générale II

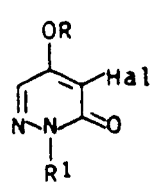

II

dans laquelle R représente un groupe alkyle et Hal un halogène, par réaction avec de l'hydrazine, en la 5-amino-6-pyridazone III

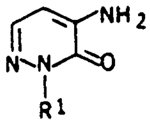

III

et on transforme ensuite celle-ci en Ia par réaction avec un composé de la formule générale IV

B-A-X IV

dans laquelle X représente halogène ou carboxylate.

**3.** Procédé de préparation de composés Ib selon la revendication I, caractérisé par le fait que l'on transforme en Ib, un dérivé de 5-amino-6-pyridazone de formule Ia, par réaction, en présence d'une base, avec un composé de la formule générale V

$R^2$-Y V

dans laquelle Y représente halogène, un groupe sulfonate ou un groupe sulfate.

**4.** Utilisation comme herbicides des dérivés de pyridazones substitués Ia et/ou Ib selon la revendication 1.

**5.** Herbicides, selon la revendication 1, contenant- un dérivé de pyridazone substitué Ia et/ou Ib et des additifs inertes.

**6.** Herbicides selon la revendication 5, contenant un dérivé de pyridazone substitué Ia et/ou Ib et d'autres herbicides et/ou constituants à action synergétique.

**7.** Procédé de lutte contre la croissance de plantes indésirables, caractérisé par le fait que l'on traite les plantes indésirables avec une quantité efficace comme herbicide d'un dérivé de pyridazone substitué Ia et/ou Ib selon la revendication 1.